# EUROPEAN PATENT APPLICATION

(11) **EP 2 444 492 A1**
(43) Date of publication of application: **25.04.2012**
(21) Application number: 10789545.0
(22) Date of filing: 17.06.2010
(51) Int. Cl.: C12N 15/09, A61K 39/395, A61P 7/02, A61P 9/00, A61P 9/10, A61P 29/00, C07K 16/28

(54) **CHICKEN-DERIVED ANTI-LOX-1 ANTIBODY**

(30) Priority: 18.06.2009 JP 2009145696
(71) Applicant: Hiroshima Bio-medical Co., Ltd., Hiroshima 739-0046 (JP)
(72) Inventor: MATSUDA Haruo, Higashi-Hiroshima-shi Hiroshima 739-0142 (JP); SAWAMURA Tatsuya, Suita-shi Osaka 565-0824 (JP)
(74) Representative: Johansson, Lars E.
(86) International application number: PCT/JP2010/060266
(87) International publication number: WO 2010/147171

(57) **Abstract**

Anti-LOX-1 antibodies having higher efficiency in action and excellent cross-reactivity are obtained. The anti-LOX-1 antibodies having higher efficiency in action and excellent cross-reactivity have been obtained, the antibodies being which specifically binds to a LOX-1 and having heavy chain CDR3 comprising one or more amino acid sequences selected from the group consisting of a) an amino acid sequence set forth in SEQ ID NO: 1, b) an amino acid sequence having 80% or more homology to the amino acid sequence a), c) an amino acid sequence having one or several amino acid deletions, substitutions, or additions in the amino acid sequence a), and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence a).

## Description

### TECHNICAL FIELD

The present invention relates to LOX-1-binding antibodies.

### BACKGROUND ART

A lectin-like oxidized LDL receptor (lectin-like oxidized LDL receptor-1: LOX-1) is a membrane protein which binds to oxidized LDL (oxidized low-density lipoprotein : oxLDL) and which evokes various cellular responses. In addition, it has been proposed that LOX-1 plays a key role as a critical cause for a cardiovascular disease or an atherosclerotic disease, etc. (Patent Document 1, Non-Patent Document 1).

According to WHO (World Health Organization), the cardiovascular disease is the most frequent cause of death in the world. It has been reported that 17.5 million people died from the cardiovascular disease, which accounts for 30% of the death causes all over the world in the 2005 statics (Non-Patent Document 2). Meanwhile, the atherosclerotic disease is a disease which makes an artery wall thick and thus decreases an intrinsic function of blood vessels. The disease causes myocardial infarction or cerebral infarction with a fatal risk. The number of death caused by such noncommunicable diseases (NCDs) is presumed to increase in a future (Non-Patent Document 3). Accordingly, the world is expecting development of a therapeutic or diagnostic agent having a novel mechanism of action against the cardiovascular disease or the atherosclerotic disease. Here, LOX-1 attracts attention as an important target.

Recently, the research and development of an antibody medicine or diagnostic agent using an antibody has been in rapid progress. Anti-LOX-1 monoclonal antibodies (anti-LOX-1 mAbs) should be applicable for a therapeutic or diagnostic agent for diseases involved with LOX-1. However, the LOX-1 CTL domain is highly conserved among mammals. Thus, the mAbs against LOX-1 are difficult to produce by immunizing the mammals with LOX-1. Due to this, there is a few examples that mammalian anti-LOX-1 mAbs have been obtained by experiments (Non-Patent Documents 4, 5, 6, and 7).

The present inventors have tried to produce anti-LOX-1 mAbs in a mammal, and have reported that they have successfully generated anti-LOX-1 mAbs having cross-reactivity to a human, a pig, cattle, and/or a rabbit (Patent Document 1). Furthermore, the present inventors have also obtained effective *in vivo* drug efficacy data on the anti-human-LOX-1 mAbs which have been administered to disease model rats having LPS-induced thrombocytopenia, an LPS-induced lung disorder, an LPS-induced inflammation, post-PTCA restenosis, or arterial thrombosis. The above was innovative research results demonstrating usefulness of the anti-LOX-1 mAbs as prophylactic, therapeutic, and/or diagnostic agents for diseases involved with LOX-1.

Meanwhile, as an effort to generate an antibody against a biomolecule that is highly conserved among mammals, it has been reported that a chicken immune system has been utilized to generate chicken anti-PrP mAbs (Non-Patent Documents 8, 9, 10). PrP (prion protein) has 80% or more homology to its amino acid sequences among mammals, so that it is difficult to produce the anti-PrP mAbs by immunizing the mammal. In view of this, the anti-PrP mAbs have been successfully generated by using a chicken in which the amino acid sequence of PrP has 40% or less homology to that of the mammals. In addition, as an effort to decrease immunogenicity in the case of administration to a human subject, a chicken-human chimeric antibody and a humanized antibody of the chicken-derived antibody have been successfully produced (Patent Documents 2, 3, and 4).

Moreover, recently, it has been getting clear that LOX-1 recognizes not only oxLDL, but also cells undergoing apoptosis, senescent erythrocytes, and inflammatory cells, etc., so that LOX-1 plays a critical role in diverse biological phenomena such as a biological defense mechanism and an inflammatory mechanism.

### PRIOR ART REFERENCE

### PATENT DOCUMENT

[Patent Document 1] WO01/064862
[Patent Document 2] JP2006-282521A
[Patent Document 3] JP2005-245337A
[Patent Document 4] JP2006-241026A

[Non-Patent Document 1] Mehta et al., Cardiovasc Res. 2006 Jan., 69(1), 36-45, Epub 2005 Dec 1.
[Non-Patent Document 2] "Cardiovascular diseases" Fact sheet N° 317 February 2007, World Health Organization.
[Non-Patent Document 3] Abegunde et al., Lancet 2007 Dec 8, 370(9603), 1929-1938.
[Non-Patent Document 4] Sawamura et al., Nature 1997 Mar 6, 386(6620), 73-77.
[Non-Patent Document 5] Chen et al., Biochem J. 2001 Apr 15, 355(Pt 2), 289-296.
[Non-Patent Document 6] Hu et al., Biochem Biophys Res Commun. 2003 Aug 8, 307(4), 1008-1012.
[Non-Patent Document 7] Delneste et al., Immunity 2002 Sep., 17(3), 353-362.
[Non-Patent Document 8] Matsuda et al., FEMS Immunol Med Microbiol. 1999 Mar., 23(3), 189-194.
[Non-Patent Document 9] Nakamura et al., Cytotechnology 2000 Mar., 32(3), 191-198.
[Non-Patent Document 10] Nakamura et al., J Vet Med Sci. 2004 Jul., 66(7), 807-814.

### SUMMARY OF THE INVENTION

### PROBLEMS TO BE SOLVED BY THE INVENTION

Unfortunately, the conventional technology as described in the above documents has had room for improvement in respect to the following points. First, there has been a demand to obtain an LOX-1-binding antibody (hereinafter, sometimes referred to as an anti-LOX-1 antibody) which reacts more efficiently with LOX-1. This is because when the anti-LOX-1 antibody is developed and sold for applications as a therapeutic agent, effects can be achieved which reduce development cost, user's financial burden and patient's physical strain by decreasing its usage. The same applies to development and sales of other applications such as a diagnostic agent.

In particular, recently in pharmaceutical industry, an antibody medicine business has attracted the most attention and has been heavily invested. In this business, how to improve antibody efficiency in action becomes a big issue. In fact, a pharmaceutical company with an emphasis on the antibody medicine and an antibody medicine venture are taking every measure and are seeking to find means for enhancing the antibody efficiency. For example, Kyowa Hakko Kirin Co., Ltd. possesses POTELLIGENT and COMPLEGENT technology, Genentech Inc. possesses Fab-fragment technology, Ablynx Inc. possesses Nanobodies technology, and Domantis Inc. possesses domain antibody technology, etc. Some of them have been proved to have effectiveness and safety, and are commercially available as an innovative new medicin which replaces existing drugs. In addition, almost every antibody medicine cannot achieve a sufficient effect by sole administration, and thus is administered by combining it with a chemotherapeutic agent. This tells a situation that an antibody having a much higher efficiency in action is sought for the antibody medicine. In view of the above, in the process of looking into the development of the antibody medicine, the present invention is required to produce anti-LOX-1 mAbs having a much higher efficiency in action.

Second, in the documents above, anti-LOX-1 mAbs have not been obtained which have cross-reactivity to human and mouse LOX-1. Mice are readily available and are easily handled, so that mice remain the most frequently used animal models for various human diseases. These animal models are important in analyzing disease manifestation and verifying effects of a therapeutic or diagnostic agent. In a similar manner, mice are also an important disease animal model for a human cardiovascular disease or atherosclerotic disease involved with LOX-1. For example, an ApoE-knockout mouse is being used as an animal model for primary hyperlipidemia [Sato et al., Atherosclerosis, 2008 Oct., 200(2), 303-309, Epub 2008 Feb 12]. As for these models, a further improvement should have been executed to obtain anti-LOX-1 mAbs exhibiting cross-reactivity to human and mouse LOX-1.

The present invention has been made in light of the above situation. It is an object of the present invention to provide a LOX-1-binding antibody having an extremely high association rate constant (ka). In addition, it is another object of the present invention to provide a LOX-1-binding antibody which binds to LOX-1 proteins derived from mammals of different species.

### MEANS FOR SOLVING THE PROBLEMS

The present invention provides a LOX-1-binding antibody which specifically binds to a LOX-1 protein and which comprises an amino acid sequence set forth in SEQ ID NO: 1 as heavy chain CDR3.

This LOX-1-binding antibody has been demonstrated in Examples below to bind to any of LOX-1 proteins derived from a human, a rabbit, and a pig. In addition, the Examples below have demonstrated that this LOX-1-binding antibody has an association rate constant (ka) for LOX-1 at 1.91 × 10⁵ M⁻¹s⁻¹.

Also, those skilled in the art can easily conceive that a reasonably similar effect can be obtained when the heavy chain CDR3 comprises one or more amino acid sequences selected from the group consisting of a) an amino acid sequence set forth in SEQ ID NO: 1, b) an amino acid sequence having 80% or more homology to the amino acid sequence set forth in SEQ ID NO: 1, c) an amino acid sequence having one or several amino acid deletions, substitutions, or additions in the amino acid sequence set forth in SEQ ID NO: 1, and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

In addition, the present invention provides a polynucleotide comprising a nucleotide sequence encoding a LOX-1-binding antibody which comprises an amino acid sequence set forth in SEQ ID NO: 1 as heavy chain CDR3 and which specifically binds to a LOX-1 protein.

This polynucleotide comprises a nucleotide sequence encoding the LOX-1-binding antibody which has been demonstrated to bind to any of LOX-1 proteins derived from a human, a rabbit, and a pig. Thus, a polynucleotide is obtained which includes a nucleotide sequence encoding a LOX-1-binding antibody having cross-reactivity to any of LOX-1 proteins derived from a human, a rabbit, and a pig. In addition, this polynucleotide comprises a nucleotide sequence encoding a LOX-1-binding antibody which has been demonstrated to exhibit an association rate constant (ka) for LOX-1 at 1.91 × 10⁵ M⁻¹s⁻¹ as described in Examples below. Thus, a polynucleotide is obtained which comprises a nucleotide sequence encoding a LOX-1-binding antibody having an extremely high association rate constant (ka) for LOX-1.

Also, those skilled in the art can easily conceive that a reasonably similar effect can be obtained when the heavy chain CDR3 comprises one or more amino acid sequences selected from the group consisting of a) an amino acid sequence set forth in SEQ ID NO: 1, b) an amino acid sequence having 80% or more homology to the amino acid sequence set forth in SEQ ID NO: 1, c) an amino acid sequence having one or several amino acid deletions, substitutions, or additions in the amino acid sequence set forth in SEQ ID NO: 1, and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

In addition, the present invention provides an inhibitor for binding of LOX-1 to oxLDL (i.e., oxLDL-binding with LOX-1), the inhibitor containing a LOX-1-binding antibody which comprises the amino acid sequence set forth in SEQ ID NO: 1 as heavy chain CDR3 and which specifically binds to a LOX-1 protein.

This binding inhibitor contains a LOX-1-binding antibody which has been demonstrated to inhibit the binding between LOX-1 and oxLDL as described in Examples below. Because of this, this binding inhibitor is preferably used as an inhibitor for binding of LOX-1 to oxLDL.

Also, those skilled in the art can easily conceive that a reasonably similar effect can be obtained when the heavy chain CDR3 comprises one or more amino acid sequences selected from the group consisting of a) an amino acid sequence set forth in SEQ ID NO: 1, b) an amino acid sequence having 80% or more homology to the amino acid sequence set forth in SEQ ID NO: 1, c) an amino acid sequence having one or several amino acid deletions, substitutions, or additions in the amino acid sequence set forth in SEQ ID NO: 1, and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

In addition, the present invention provides an inhibitor for uptake of oxLDL into LOX-1-expressing cells, the inhibitor containing a LOX-1-binding antibody which comprises the amino acid sequence set forth in SEQ ID NO: 1 as heavy chain CDR3 and which specifically binds to a LOX-1 protein.

This uptake inhibitor contains a LOX-1-binding antibody which has been demonstrated to inhibit uptake of oxLDL into LOX-1-expressing cells. Because of this, this uptake inhibitor is preferably used as an inhibitor for uptake of oxLDL into LOX-1-expressing cells.

Also, those skilled in the art can easily conceive that a reasonably similar effect can be obtained when the heavy chain CDR3 comprises one or more amino acid sequences selected from the group consisting of a) an amino acid sequence set forth in SEQ ID NO: 1, b) an amino acid sequence having 80% or more homology to the amino acid sequence set forth in SEQ ID NO: 1, c) an amino acid sequence having one or several amino acid deletions, substitutions, or additions in the amino acid sequence set forth in SEQ ID NO: 1, and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

In addition, the present invention provides a therapeutic agent containing a LOX-1-binding antibody which comprises the amino acid sequence set forth in SEQ ID NO: 1 and which specifically binds to a LOX-1 protein. The agent is used for a cardiovascular disease, an atherosclerotic disease, thrombosis, thrombocytopenia, a systemic inflammatory response syndrome, an inflammation, or intimal hyperplasia of mammals.

A LOX-1-binding antibody has been demonstrated to have a therapeutic effect on a cardiovascular disease, an atherosclerotic disease, thrombosis, thrombocytopenia, a systemic inflammatory response syndrome, an inflammation, or intimal hyperplasia of mammals in WO01/064862; Inoue et al., Circ Res. 2005 Jul 22, 97(2), 176-184; Hinagata et al., Cardiovasc Res. 2006 Jan., 69(1), 263-271; and Honjo et al., Proc Natl Acad Sci USA 2003 Feb 4, 100(3), 1274-1279. Because of this, this therapeutic agent is preferably used as a therapeutic agent for a cardiovascular disease, an atherosclerotic disease, thrombosis, thrombocytopenia, a systemic inflammatory response syndrome, an inflammation, or intimal hyperplasia of mammals.

Also, those skilled in the art can easily conceive that a reasonably similar effect can be obtained when the heavy chain CDR3 comprises one or more amino acid sequences selected from the group consisting of a) an amino acid sequence set forth in SEQ ID NO: 1, b) an amino acid sequence having 80% or more homology to the amino acid sequence set forth in SEQ ID NO: 1, c) an amino acid sequence having one or several amino acid deletions, substitutions, or additions in the amino acid sequence set forth in SEQ ID NO: 1, and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

In addition, the present invention provides a diagnostic agent containing a LOX-1-binding antibody which comprises the amino acid sequence set forth in SEQ ID NO: 1 and which specifically binds to a LOX-1 protein. The agent is used for a cardiovascular disease, an atherosclerotic disease, thrombosis, thrombocytopenia, a systemic inflammatory response syndrome, an inflammation, or intimal hyperplasia of mammals.

A LOX-1-binding antibody has been demonstrated to have a therapeutic effect on a cardiovascular disease, an atherosclerotic disease, thrombosis, thrombocytopenia, a systemic inflammatory response syndrome, an inflammation, or intimal hyperplasia of mammals in WO01/064862; Inoue et al., Circ Res. 2005 Jul 22, 97(2), 176-184; Hinagata et al., Cardiovasc Res. 2006 Jan., 69(1), 263-271; and Honjo al., Proc Natl Acad Sci USA 2003 Feb 4, 100(3), 1274-1279. In addition, the diagnosis of the above diseases seems to become possible by measuring and comparing the levels of LOX-1 expression in a standard cell, a test cell, a test serum, or a pathologic cell of any of the above diseases in accordance with the binding mode of this diagnostic agent. Because of this, this diagnostic agent is preferably used as a diagnostic agent for a cardiovascular disease, an atherosclerotic disease, thrombosis, thrombocytopenia, a systemic inflammatory response syndrome, an inflammation, or intimal hyperplasia of mammals.

Also, those skilled in the art can easily conceive that a reasonably similar effect can be obtained when the heavy chain CDR3 comprises one or more amino acid sequences selected from the group consisting of a) an amino acid sequence set forth in SEQ ID NO: 1, b) an amino acid sequence having 80% or more homology to the amino acid sequence set forth in SEQ ID NO: 1, c) an amino acid sequence having one or several amino acid deletions, substitutions, or additions in the amino acid sequence set forth in SEQ ID NO: 1, and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

The present invention provides a LOX-1-binding antibody which specifically binds to a LOX-1 protein and which comprises an amino acid sequence set forth in SEQ ID NO: 4 as heavy chain CDR3.

This LOX-1-binding antibody has been demonstrated to bind to any of LOX-1 proteins derived from a human, a mouse, a rabbit, and a pig. In addition, this LOX-1-binding antibody has been obtained using a method similar to that for a LOX-1-binding antibody which has been demonstrated to exhibit an association rate constant (ka) for LOX-1 at 1.91 × 10⁵ M⁻¹s⁻¹ as described in Examples below. Thus, a LOX-1-binding antibody can be obtained which has an extremely high association rate constant (ka) for LOX-1.

Also, those skilled in the art can easily conceive that a reasonably similar effect can be obtained when the heavy chain CDR3 comprises one or more amino acid sequences selected from the group consisting of m) an amino acid sequence set forth in SEQ ID NO: 4, n) an amino acid sequence having 80% or more homology to the amino acid sequence set forth in SEQ ID NO: 4, o) an amino acid sequence having one or several amino acid deletions, substitutions, or additions in the amino acid sequence set forth in SEQ ID NO: 4, and p) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 4.

The present invention provides a LOX-1-binding antibody which specifically binds to a LOX-1 protein and which comprises an amino acid sequence set forth in SEQ ID NO: 5 as heavy chain CDR3.

This LOX-1-binding antibody has been demonstrated to bind to any of LOX-1 proteins derived from a human and a mouse. In addition, this LOX-1-binding antibody has been obtained using a method similar to that for a LOX-1-binding antibody which has been demonstrated to exhibit an association rate constant (ka) for LOX-1 at 1.91 × 10⁵ M⁻¹s⁻¹ as described in Examples below. Thus, a LOX-1-binding antibody can be obtained which has an extremely high association rate constant (ka) for LOX-1.

Also, those skilled in the art can easily conceive that a reasonably similar effect can be obtained when the heavy chain CDR3 comprises one or more amino acid sequences selected from the group consisting of q) an amino acid sequence set forth in SEQ ID NO: 5, r) an amino acid sequence having 80% or more homology to the amino acid sequence set forth in SEQ ID NO: 5, s) an amino acid sequence having one or several amino acid deletions, substitutions, or additions in the amino acid sequence set forth in SEQ ID NO: 5, and t) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 5.

The present invention provides a LOX-1-binding antibody which specifically binds to a LOX-1 protein and which comprises an amino acid sequence set forth in SEQ ID NO: 6 as heavy chain CDR3.

This LOX-1-binding antibody has been demonstrated to bind to any of LOX-1 proteins derived from a human, a rabbit, and a pig. In addition, this LOX-1-binding antibody has been obtained using a method similar to that for the LOX-1-binding antibody which has been demonstrated to exhibit an association rate constant (ka) for LOX-1 at 1.91 × 10⁵ M⁻¹s⁻¹ as described in Examples below. Thus, a LOX-1-binding antibody can be obtained which has an extremely high association rate constant (ka) for LOX-1.

Also, those skilled in the art can easily conceive that a reasonably similar effect can be obtained when the heavy chain CDR3 comprises one or more amino acid sequences selected from the group consisting of u) an amino acid sequence set forth in SEQ ID NO: 6, v) an amino acid sequence having 80% or more homology to the amino acid sequence set forth in SEQ ID NO: 6, w) an amino acid sequence having one or several amino acid deletions, substitutions, or additions in the amino acid sequence set forth in SEQ ID NO: 6, and x) an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 6.

The present invention provides a LOX-1-binding antibody which specifically binds to a LOX-1 protein and which comprises an amino acid sequence set forth in SEQ ID NO: 7 as heavy chain CDR3.

This LOX-1-binding antibody has been demonstrated to bind to any of LOX-1 proteins derived from a human and a pig. In addition, this LOX-1-binding antibody has been obtained using a method similar to that for a LOX-1-binding antibody which has been demonstrated to exhibit an association rate constant (ka) for LOX-1 at 1.91 × 10⁵ M⁻¹s⁻¹ as described in Examples below. Thus, a LOX-1-binding antibody can be obtained which has an extremely high association rate constant (ka) for LOX-1.

Also, those skilled in the art can easily conceive that a reasonably similar effect can be obtained when the heavy chain CDR3 comprises one or more amino acid sequences selected from the group consisting of y) an amino acid sequence set forth in SEQ ID NO: 7, z) an amino acid sequence having 80% or more homology to the amino acid sequence set forth in SEQ ID NO: 7, a') an amino acid sequence having one or several amino acid deletions, substitutions, or additions in the amino acid sequence set forth in SEQ ID NO: 7, and b') an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 7.

### ADVANTAGEOUS EFFECTS OF THE INVENTION

Since LOX-1-binding antibodies have particular amino acid sequences of CDR3 according to the present invention, the antibodies can be obtained which have an excellent LOX-1-binding property. In addition, LOX-1-binding antibodies having broad cross-reactivity can be obtained because the antibodies possess the particular amino acid sequences of CDR3.

### BRIEF DESCRIPTION OF THE DRAWINGS

[FIG. 1] FIG. 1 is a conceptual diagram of LOX-1.
[FIG. 2] FIG.2 is a picture and a graph showing a) a SDS-PAGE profile of the recombinant LOX-1 and b) ELISA results of measuring the binding mode of rhLOX-1 toward oxLDL, LDL, and BSA.
[FIG. 3] FIG. 3 is graphs showing A) results of measuring the binding mode of recombinant chicken antibodies toward recombinant LOX-1 proteins and B) results of measuring cross-reactivity of nine recombinant chicken antibodies to LOX-1 proteins derived from four different species.
[FIG. 4] FIG. 4 is pictures of Western blotting detecting a recombinant chicken antibody and a chicken-human chimeric antibody.
[FIG. 5] FIG.5 is graphs showing results of measuring a rate of inhibiting binding of oxLDL to LOX-1 by each of a recombinant chicken antibody and a chicken-human chimeric antibody.
[FIG. 6] FIG. 6 is graphs and photographs showing A) results of measuring the binding modes of chicken-human chimeric antibodies toward LOX-1-expressing CHO cells and B) results of measuring the blocking activity during addition of oxLDL.
[FIG 7] FIG. 7 is a chart showing results of measuring the binding mode between LOX-1 and anti-LOX-1 mAbs as determined by BIACORE analysis.

### MODES FOR CARRYING OUT THE INVENTION

### <History of the Present Invention>

The present inventors have been conducting research which analyzes LOX-1 protein functions and intends to improve effects of anti-LOX-1 antibodies so as to develop a therapeutic and/or diagnostic agent for diseases. For example, WO01/064862 discloses that: anti-human-LOX-1 mAbs have been generated by immunizing a human-antibody-producing transgenic mouse; the generated anti-human-LOX-1 mAbs have been administered to rat models for diseases such as LPS-induced thrombocytopenia, an LPS-induced lung disorder, an LPS-induced inflammation, post-PTCA restenosis, or arterial thrombogenesis; and the effective *in vivo* drug efficacy data have been obtained. However, in the case of development of a therapeutic agent, applicants must receive an authority's approval for commercialization by spending a limited amount of budget and time to undergo non-clinical and clinical trials. In order to achieve this goal, an anti-LOX-1 antibody having a further potent effect should have been attained. In addition, in order to make more efficient diagnosis, another anti-LOX-1 antibody having a potent effect has been sought for applications as a diagnostic agent. Furthermore, there has been a demand for obtaining an anti-LOX-1 antibody having cross-reactivity to LOX-1 proteins derived form a human and a mouse which is useful as a disease model animal.

Meanwhile, the present inventors have successfully generated anti-PrP chicken mAbs, which have been difficult to produce in mammals, by utilizing a chicken as disclosed in Nakamura et al., J Immunol Methods, 2003 Sep., 280(1-2), 157-164. As described in such an example, a chicken is phylogenetically diverged from mammals, so that the chicken possesses a potential to generate an antibody which cannot be produced in the mammals.

Accordingly, the present inventors have examined and tried to obtain an anti-LOX-1 antibody having a more advantageous effect by utilizing a chicken. As a result, chicken-derived anti-LOX-1 mAbs have been generated, and anti-LOX-1 mAbs having an extremely high association rate constant (ka) have been further successfully generated by optimizing a phage display procedure. Moreover, cross-reactivity of the generated anti-LOX-1 mAbs has been investigated, and anti-LOX-1 mAbs have then been obtained which have cross-reactivity to one or two or all of pig LOX-1, rabbit LOX-1, and mouse LOX-1. Finally, the present invention has been completed.

### <Description of Terms>

In the specification of the present application and the Claims, meanings of various terms are defined as follows.

### (1) LOX-1 (lectin-like oxidized LDL receptor or lectin-like oxidized LDL receptor-1)

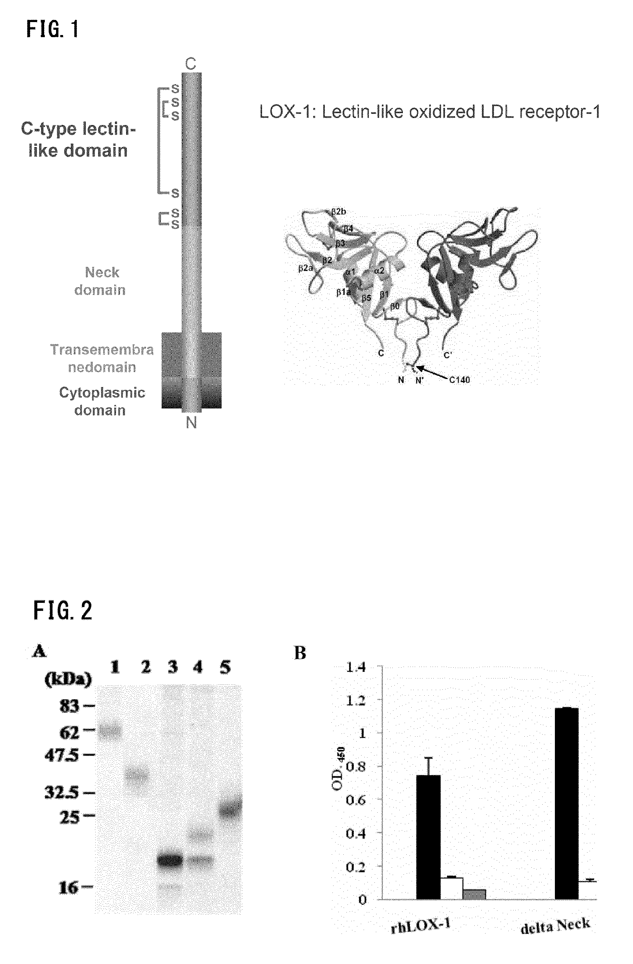

LOX-1 (sometimes herein designated as a LOX-1 protein) is a membrane protein which binds to oxLDL and evokes various cellular responses. In addition, typical LOX-1 is an about 50-kDa protein consisting of 273 amino acid residues and constitutes a protein having one transmembrane domain, what is called a type-II membrane protein, including an N-terminal portion in cytoplasm and a C-terminal portion outside of cell (FIG. 1). LOX-1 is divided into 4 domains including, from the N-terminus, a cytoplasmic domain, a transmembrane domain, a Neck domain, and a lectin-like domain (a CTL domain). The CTL domain has homology to a sugar chain recognition site of the type C lectin family. The positions of six cysteine residues which are well conserved among these proteins completely remain the same. The CTL domain is important in LOX-1 functions. The C-terminal residue and an arginine residue in this domain are indispensable for the binding to oxLDL [Chen et al., Biochem J. 2001 Apr 15, 355(Pt 2), 289-296; Ohki et al., Structure 2005 Jun, 13(6), 905-917]. LOX-1 has high homology to NKRP1 expressed on NK cells, in particular, among the lectin family. NKRP1 is a protein essential in activation of an NK cell when the NK cell recognizes and kills a tumor cell. From the beginning, LOX-1 may seem to have not only a function of recognizing and uptaking oxLDL, but also a function of activating endothelial cells and related functions thereof. Then, the following data have been revealed in respect to LOX-1: LOX-1 recognizes cells undergoing apoptosis, senescent erythrocytes, and inflammatory cells; LOX-1 plays a critical role in important biological phenomena such as a biological defense mechanism and an inflammatory mechanism; and its expression is dynamically regulated by a stimulus under various conditions. Recently, research on the pathophysiological significance of LOX-1 has been in progress among a wide variety of fields. In the course of such research, it is still being elucidated that LOX-1 is deeply involved in pathogenesis of various diseases.

### (2) oxLDL

oxLDL is a substance which is generated by further oxidizing an LDL cholesterol, a bad cholesterol. The oxLDL is considered to be a risk factor which is involved in any step of from the onset to the maturation of an atherosclerotic disease as revealed by *in vitro* research utilizing arteriosclerosis-related cells and studies utilizing disease model animals.

### (3) Heavy Chain

A heavy chain is a key component of an antibody, and is typically linked to a light chain via a disulfide bond and a non-covalent bond. In an N-terminal domain of the heavy chain, a region whose amino acid sequence varies among antibodies of the same species and class, what is called a variable region (V_{H}), is present. It is generally known that the V_{H} contributes largely to the specificity and affinity to an antigen. For example, a V_{H} gene has been extracted and amplified from a spleen of a mouse immunized with lysozyme. Next, the only V_{H} has been expressed solely. Then, it has been reported that the V_{H} fragment has remarkably retained the affinity to the antigen [Ward et al., Nature 1989 Oct 12, 341(6242), 544-546]. In addition, among antibodies of a camel, it has been reported that there is a molecular species in which heavy chains are present as a dimer without a light chain [Wolfson W, Chem Biol. 2006 Dec., 13(12), 1243-1244].

### (4) CDR (complementarity determining region)

CDR is a region which has a binding site thereon by directly and practically contacting an antigen on Fv (i.e., variable regions, which include a V region of a heavy chain (V_{H}) and a V region of a light chain (V_{L})) of an antibody molecule. The heavy and light chains each have CDR1, CDR2, and CDR3, all of which contain about 5 to 10 amino acid residues. Generally speaking, the CDR3 most contributes to the binding. The CDRs are regions which define specificity to an antigen. Accordingly, their amino acid sequences largely vary among antibodies. Thus, the CDRs are also referred to as hypervariable regions. Fv regions excluding the CDRs are referred to as framework regions (FR), including FR1, FR2, FR3, and FR4, which are relatively well conserved among antibodies [Kabat et al., "Sequence of Proteins of Immunological Interest", US Dept. Health and Human Services, 1983].

Hereinafter, embodiments of the present invention are described in detail. It is notable that in order to avoid redundancy, similar contents are not repeated.

### <Embodiment 1 : Production of LOX-1-binding antibody containing, as heavy chain CDR3, amino acid sequence set forth in SEQ ID NO: 1 or homologous sequence thereof>

LOX-1-binding antibodies according to this embodiment include a LOX-1-binding antibody having, as heavy chain CDR3, an amino acid sequence set forth in SEQ ID NO: 1. Examples below demonstrate that this LOX-1-binding antibody exhibits an association rate constant (ka) for LOX-1 at 1.91 × 10⁵ M⁻¹s⁻¹. Thus, the antibody is a LOX-1-binding antibody having an extremely high association rate constant (ka) for LOX-1. In addition, this LOX-1-binding antibody has been demonstrated to inhibit the binding of LOX-1 to oxLDL. Accordingly, this LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, pig, and rabbit LOX-1. In view of this, this LOX-1-binding antibody seems to be suitably used for applications as a therapeutic and/or diagnostic agent for diseases such as a cardiovascular disease and an atherosclerotic disease which are involved with LOX-1.

Here, the LOX-1-binding antibodies include a LOX-1-binding antibody whose heavy chain CDR3 has 80% or more homology to the amino acid sequence set forth in SEQ ID NO: 1. In addition, the heavy chain CDR3 has preferably 85% or more homology to the amino acid sequence set forth in SEQ ID NO: 1, more preferably 90% or more, still more preferably 95% or more, and still more preferably 98% or more. This is because if the heavy chain CDR3 contains an amino acid sequence having higher homology to the sequence set forth in SEQ ID NO: 1, functions can be achieved which approach those of LOX-1-binding antibody whose heavy chain CDR3 has the amino acid sequence set forth in SEQ ID NO: 1, the antibody having an extremely high association rate constant (ka) for LOX-1 as evidenced by Examples below.

In addition, the LOX-1-binding antibodies include a LOX-1-binding antibody whose heavy chain CDR3 has one or several amino acid deletions, substitutions, or additions in the amino acid sequence set forth in SEQ ID NO: 1. Here, the heavy chain CDR3 contains an amino acid sequence having preferably one to three amino acid deletions, substitutions, or additions in the amino acid sequence set forth in SEQ ID NO: 1, more preferably one or two amino acid deletions, substitutions, or additions, and still more preferably one amino acid deletion, substitution, or addition. This is because if the heavy chain CDR3 contains an amino acid sequence having higher homology to the sequence set forth in SEQ ID NO: 1, it will have a similar function to the LOX-1-binding antibody whose heavy chain CDR3 has the amino acid sequence set forth in SEQ ID NO: 1, the antibody having an extremely high association rate constant (ka) for LOX-1 as evidenced by Examples below.

In addition, the LOX-1-binding antibodies include a LOX-1-binding antibody whose heavy chain CDR3 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 1.

In addition, the LOX-1-binding antibodies include a LOX-1-binding antibody whose heavy chain CDR1 has the amino acid sequence set forth in SEQ ID NO: 2. Examples below demonstrate that the LOX-1-binding antibody exhibits an association rate constant (ka) for LOX-1 at 1.91 × 10⁵ M⁻¹s⁻¹. Thus, the antibody is a LOX-1-binding antibody having an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, pig, and rabbit LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR1 has 90% or more homology to the amino acid sequence set forth in SEQ ID NO: 2. Here, the heavy chain CDR1 has preferably 95% or more homology to the amino acid sequence set forth in SEQ ID NO: 2, and more preferably 98% or more. This is because if the heavy chain CDR1 contains an amino acid sequence having higher homology to the sequence set forth in SEQ ID NO: 2, functions can be achieved which approach those of the LOX-1-binding antibody whose heavy chain CDR1 has the amino acid sequence set forth in SEQ ID NO: 2, the antibody having an extremely high association rate constant (ka) for LOX-1 as evidenced by Examples below.

In addition, the LOX-1-binding antibodies include a LOX-1-binding antibody whose heavy chain CDR1 has one amino acid deletion, substitution, or addition in the amino acid sequence set forth in SEQ ID NO: 1.

In addition, the LOX-1-binding antibodies include a LOX-1-binding antibody whose heavy chain CDR1 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 2.

In addition, the LOX-1-binding antibodies include a LOX-1-binding antibody whose heavy chain CDR2 has the amino acid sequence set forth in SEQ ID NO: 3. In this case, Examples below demonstrate that the LOX-1-binding antibody exhibits an association rate constant (ka) for LOX-1 at 1.91 × 10⁵ M⁻¹s⁻¹. Thus, the antibody is a LOX-1-binding antibody having an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, pig, and rabbit LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR2 has 85% or more homology to the amino acid sequence set forth in SEQ ID NO: 3. Here, the heavy chain CDR2 has preferably 90% or more homology to the amino acid sequence set forth in SEQ ID NO: 3, more preferably 95% or more, and still more preferably 98% or more. This is because if the heavy chain CDR2 contains an amino acid sequence having higher homology to the sequence set forth in SEQ ID NO: 3, functions can be achieved which approach those of the LOX-1-binding antibody whose heavy chain CDR2 has the amino acid sequence set forth in SEQ ID NO: 3, the antibody having an extremely high association rate constant (ka) for LOX-1 as evidenced by Examples below.

In addition, the LOX-1-binding antibodies include a LOX-1-binding antibody whose heavy chain CDR2 has one or several amino acid deletions, substitutions, or additions in the amino acid sequence set forth in SEQ ID NO: 3. Here, the heavy chain CDR2 contains an amino acid sequence having preferably one to three amino acid deletions, substitutions, or additions in the amino acid sequence set forth in SEQ ID NO: 3, more preferably one or two amino acid deletions, substitutions, or additions, and still more preferably one amino acid deletion, substitution, or addition. This is because if the heavy chain CDR2 contains an amino acid sequence having higher homology to the sequence set forth in SEQ ID NO: 3, functions can be achieved which approach those of the LOX-1-binding antibody whose heavy chain CDR2 has the amino acid sequence set forth in SEQ ID NO: 3, the antibody having an extremely high association rate constant (ka) for LOX-1 as evidenced by Examples below.

In addition, the LOX-1-binding antibodies include a LOX-1-binding antibody whose heavy chain CDR2 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 3.

Also, the LOX-1-binding antibodies include a LOX-1-binding antibody which binds to LOX-1 proteins derived from mammals of different species. The mammals of different species herein refer to a human and one or more species selected from a mouse, a rat, a rabbit, a pig, a sheep, cattle, a horse, a cat, a dog, a monkey, and a chimpanzee. This is because when a therapeutic and/or diagnostic agent for a human disease is developed, the following mammals can be used as typical disease model animals, the mammals including a mouse, a rat, a rabbit, a pig, a sheep, cattle, a horse, a cat, a dog, a monkey, and a chimpanzee. In addition, the mammals are a human and preferably any one or more species selected from a mouse, a rabbit, and a pig. This is because when a therapeutic and/or diagnostic agent for a disease such as a cardiovascular disease and an atherosclerotic disease is developed, a mouse, a rabbit, and a pig have been already generally utilized as model animals.

Furthermore, the mammals are, particularly preferably, a human, a rabbit, and a pig. This is because a LOX-1-binding antibody which binds to LOX-1 proteins derived from a human, a rabbit, and a pig has been actually obtained in Examples below and the obtained antibody against the LOX-1 proteins has exhibited an extremely high association rate constant (ka) for LOX-1.

As used herein, the term "homology" refers to a ratio of the number of identical amino acids between two or among a plurality of amino acid sequences to the total number of amino acids as calculated by using a known process. Before the calculation of the ratio, amino acid sequences selected from the group of amino acid sequences compared are made to be aligned. If the above ratio is required to be optimized, gaps are inserted in some portions of the amino acid sequences. In addition, any conservative substitution is not considered identical. Also, the term means a ratio of the number of identical amino acids to the total number of amino acid residues including overlapping amino acids while keeping the optimal alignment. An alignment method, a ratio calculation process, and a related computer program are well known in the art. A common sequence analysis program (e.g., GENETYX, GeneChip Sequence Analysis) can be used for determination.

As used herein, the term "cross-reactivity" generally refers to a characteristic in which a certain antibody has a significant binding affinity to any of two or more antigens having a similar structure. The antigen having a similar structure herein includes proteins having high homology.

As used herein, the term "heavy chain CDR3" refers to a region which is present in a CDR of an antibody heavy chain and which contacts an antigen and has a binding site thereon. In general, heavy chain CDRs include heavy chain CDR1, heavy chain CDR2, and heavy chain CDR3. Among them, the heavy chain CDR3 most contributes to the binding. There are several reports regarding definitions of CDR and procedures for determining the position of CDR. Any of these definitions and procedures can be adopted. For example, the definition of Kabat [Sequences of proteins of immunological interest, 5th ed., U.S. Department of Health and Human Services, 1991] or the definition of Chothia [Chothia et al., J. Mol. Biol., 1987, 196, 901-917] can be adopted. In the specification of the present application, the definition of Kabat is adopted as a preferable definition. However, it is not necessary to be limited to this definition. In some cases, the definitions of both Kabat and Chothia are taken into account for the determination. For example, a CDR region having overlapping definitions or a CDR region encompassing the respective definitions can be defined as a CDR. As a specific example of such a procedure, there is a compromised definition between Kabat and Chothia, which is a procedure developed using Oxford Molecular's AbM antibody modeling software by Martin et al. [Proc. Natl. Acad. Sci. USA, 1989, 86, 9268-9272].

As used herein, the term "hybridize" refers to a state in which a certain nucleotide binds complementarily to another nucleotide via hydrogen bonds, etc., while not binding to a control nucleotide. The certain nucleotide is not necessarily specific to all the other nucleotides, but may have some specificity depending on its usage.

As used herein, the term "stringent condition" can include, but is not limited to, for example a condition using, during hybridization, a denaturing agent such as formamide (e.g., 50% (v/v) formamide), 0.1% bovine serum albumin/0.1% Ficoll/0.1% polyvinylpyrrolidone/50 mM sodium phosphate buffer at pH 6.5, 750 mM sodium chloride, and 75 mM sodium citrate at 42°C, or can include an appropriately modified condition thereof. In such a condition, a polynucleotide having higher homology should be effectively obtained with an increased temperature. It is notable that multiple factors affecting hybridization stringency are considered, the factors including a temperature, a salt concentration, and the like. Those skilled in the art can appropriately select these factors and the hybridization can achieve similar stringency. The "stringency" can be empirically determined depending on, in general, a probe length, a washing temperature, and a salt concentration. Generally speaking, as the probe becomes longer, a temperature for suitable annealing increases. As the probe becomes shorter, the temperature decreases. Hybridization, in general, depends on the ability of a denatured DNA to reanneal with a complementary strand when the strand is present in the vicinity of its melting point and at a temperature lower than the melting point. As desired homology between a probe and a sequence capable of hybridizing with the probe becomes higher, the applicable relative temperature increases. As a result, in the case of a higher temperature, the reaction condition becomes more stringent. In the case of a lower temperature, the stringency decreases. Furthermore, the stringency is inversely proportional to the salt concentration [Ausubel et al., Current Protocols in Molecular Biology, Wiley Interscience Publishers, (1995)].

As used herein, the term "binding" means a link between substances. The link may be either a covalent bond or a non-covalent bond. Examples of the link include an ion bond, a hydrogen bond, a hydrophobic interaction, and a hydrophilic interaction.

When an amino acid residue of the LOX-1-binding antibody is herein modified, the residue is preferably mutated and substituted by another amino acid whose side chain characteristics are conserved. Examples of the characteristics of the amino acid side chain can include hydrophobic amino acids (e.g., A, I, L, M, F, P, W, Y, V), hydrophilic amino acids (e.g., R, D, N, C, E, Q, G, H, K, S, T), amino acids having an aliphatic side chain (e.g., G, A, V, L, I, P), amino acids having a hydroxy-containing side chain (e.g., S, T, Y), amino acids having a sulfur-containing side chain (e.g., C, M), amino acids having a carboxylic-acid-containing or amide-containing side chain (e.g., D, N, E, Q), amino acids containing a base-containing side chain (e.g., R, K, H), and amino acids containing an aromatic side chain (e.g., H, F, Y, W)(the respective letters between parentheses denote one-letter abbreviations of amino acids). Substitutions of an amino acid by amino acids within each group are generally referred to as a conservative substitution. It has been already known that a polypeptide having its amino acid sequence modified by one or more amino acid deletions, additions, or substitutions by other amino acids can maintain its biological activity [Mark et al., Proc Natl Acad Sci U S A. 1984 Sep., 81(18), 5662-5666; Zoller et al., Nucleic Acids Res. 1982 Oct 25, 10(20), 6487-6500; and Wang et al., Science 1984 Jun 29, 224(4656), 1431-1433].

The LOX-1-binding antibody herein may be a recombinant protein produced in cells of a human or other mammals (e.g., a rat, a mouse, a rabbit, a chicken, cattle, a monkey, a pig, a horse, a sheep, a goat, a dog, a cat, a guinea pig, a hamster) having a polynucleotide encoding the LOX-1-binding antibody, a vector containing the polynucleotide encoding the LOX-1-binding antibody, or a vector containing a portion of the polynucleotide encoding the LOX-1-binding antibody. Examples of mammalian cells include COS-7 monkey cells, Vero cells, CHO Chinese hamster cells (CHO cells), dhfr-deficient CHO Chinese hamster cells (CHO (dhfr) cells), murine L cells, murine AtT-20 cells, murine myeloma cells, rat GH3 cells, human FL cells, human HEK293 cells, and the like. Alternatively, the antibody may be a recombinant protein produced in Escherichia, Bacillus, yeast, or insect cells. Since the handling is easiest and the cost is lowest among them, Escherichia is preferable as cells used for production.

In addition, examples of the above vector which can be used include E. coli-derived plasmids (e.g., pBR322, pBR325, pUC12, pUC13), Bacillus-subtilis-derived plasmids (e.g., pUB110, pTP5, pC194), yeast-derived plasmids (e.g., pSH 19, pSH 15), bacteriophages (e.g., a λ phage), animal viruses (e.g., a retrovirus, a vaccinia virus), a baculovirus, pA1-11, pXT1, pRc/CMV, pRc/RSV, pcDNAI/Neo, and the like.

Also, the above polynucleotide or vector can be introduced into cells and the antibody can be produced in accordance with a known method in the art. Examples of a method which can be used for introducing the polynucleotide or vector encoding the antibody into a cell include a calcium phosphate method, lipofection, electroporation, an adenovirus-mediated method, a retrovirus-mediated method, microinjection, and the like ["Genetic Engineering Handbook", 4th Edition, YODOSHA CO., LTD. (2003), 152-179]. A process for producing an antibody by using cells can employ methods described in, for example, "Protein Experiment Handbook", YODOSHA CO., LTD. (2003), 128-142 or Shimamoto et al., Biologicals 2005 Sep., 33(3), 169-174. In addition, the LOX-1-binding antibody may be a protein which is chemically synthesized or synthesized using a cell-free translation system.

In addition, the antibody can be purified from antibody-producing cells by using a known method in the art. Examples of a method for purifying an antibody include ammonium sulfate precipitation or ethanol precipitation, Protein A chromatography, Protein G chromatography, gel filtration chromatography, anion- or cation-exchange chromatography, phosphocellulose chromatography, hydrophobic interaction chromatography, affinity chromatography, hydroxylapatite chromatography, lectin chromatography, and the like ["Protein Experiment Handbook", YODOSHA CO., LTD. (2003), 27-52].

As used herein, the term "antibody" refers to a molecule which can be specifically bound to a specific epitope localized on an antigen, and the term includes a polyclonal antibody and a monoclonal antibody. In addition, the antibody can exist as various forms. Examples of the forms can include Fv, Fab, F(ab')₂, Fab', a diabody, a single-chain antibody (e.g., scFv, dsFv), a CDR-containing peptide, a multivalent antibody (e.g., a divalent antibody), a chimeric antibody, a humanized antibody, a human antibody, and the like. Also, the forms having a low-molecular-weight antibody or sugar-chain-modified antibody combined with a chemically synthesized existing pharmaceutical agent or pharmaceutical formulation may be allowed. In order to decrease immunogenicity when the antibody is used as a therapeutic agent, it is preferable for the antibody to have a higher proportion of human-derived amino acid sequences. Specifically, the antibody is preferably a chimeric antibody with human-derived regions, more preferably a humanized antibody, and most preferably a human antibody. In addition, in order to decrease immunogenicity or increase stability when the antibody is used as a therapeutic agent, it is preferable for the antibody to be a lower-molecular-weight molecule as long as the antibody possesses desired functions. Furthermore, as long as the antibody possesses the desired functions, the antibody may be replaced by an aptamer made of nucleic acid containing RNA or DNA.

As used herein, a polyclonal antibody can be generated by administering an immunogen containing a target antigen to a mammal (e.g., a rat, a mouse, a rabbit, a chicken, cattle, a monkey, a pig, a horse, a sheep, a goat, a dog, a cat, a guinea pig, a hamster) so as to induce production of a serum containing an antigen-specific polyclonal antibody. Administration of the immunogen may require coinjection of one or more immunizing agents and an adjuvant as desired. The adjuvant may be used for enhancing an immune response. Examples of the adjuvant include (complete or incomplete) Freund adjuvant, a mineral gel (e.g., aluminum hydroxide), a surfactant (e.g., lysolecithin, pluronic polyol, a polyanion, a peptide, oil emulsion, keyhole limpet hemocyanin, dinitrophenol), and a potentially useful human adjuvant (e.g., Bacille Calmette-Guerin (BCG) or Corynebacterium parvum). In addition, the examples include MPL-TDM adjuvant (monophosphoryl lipid A, synthetic trehalosedicorynomycolate) as well. An immunization protocol is publicly known in the art. Any method for inducing an immune response in a selected host animal may be carried out ["Protein Experiment Handbook", YODOSHA CO., LTD. (2003), 86-91].

As used herein, the term "monoclonal antibody" refers to an antibody collected from a substantially uniform antibody population. That is, individual antibodies constituting a population are identical except the antibodies having mutations that can be present in a small number of cases and that can naturally occur. A monoclonal antibody is highly specific, and corresponds to one antigen site. Further, the monoclonal antibody is distinct from a typical polyclonal antibody including different antibodies corresponding to different epitopes (antigen determinant). Each monoclonal antibody corresponds to a single epitope of an antigen. In addition to its specificity, the monoclonal antibody is useful from a viewpoint of synthesizing the antibody by a hybridoma culture without having contamination of other immunoglobulins. The modifier "monoclonal" indicates a feature of an antibody which has been obtained from a substantially uniform antibody population, but does not mean that the antibody has to be produced by any particular method. For example, the monoclonal antibody described herein can be produced by a method similar to a hybridoma method disclosed in Kohler G and Milstein C., Nature 1975 Aug 7, 256 (5517), 495-497. Alternatively, the monoclonal antibody used in the present invention can be produced by a method similar to a recombinant method disclosed in U.S. Patent No. 4816567. In addition, the monoclonal antibody used herein can be isolated from a phage antibody library by a method similar to technology described in Clackson et al., Nature 1991 Aug 15, 352 (6336), 624-628 or Marks et al., J Mol Biol. 1991 Dec 5, 222(3), 581-597. In addition, the antibody can be generated by a general production procedure disclosed in "Protein Experiment Handbook", YODOSHA CO., LTD. (2003), 92-96. Also, the monoclonal antibody used herein is preferably generated by a procedure described in Examples below.

In addition, the antibody may be affinity-matured by using the above existing selection and/or a method for inducing a mutation. An affinity-matured antibody has preferably 5 times higher affinity than a starting antibody, more preferably 10 times higher affinity, and still more preferably 20 or 30 times higher affinity. For example, biopanning utilizing an antibody phage library can be used. A typical manipulation of this method includes the steps of: reacting an immobilized target protein with an antibody phage library; removing an unbound phage antibody by washing; eluting a bound phage antibody; infecting E. coli with the bound phage antibody; and amplifying the phage antibody. Repeating the above steps several times allows a phage antibody specific to the target protein to be obtained ["Antibody Experiment Manual", Revised Version, YODOSHA CO., LTD. (2008), 211-221].

Meanwhile, Fv is a minimum antibody fragment containing a complete antigen-recognition and antigen-binding site. This Fv region is formed of a dimer between variable domains of one heavy chain and one light chain which have tight non-covalent bonds. Using this arrangement, three CDRs of the respective variable domains interact with one another to form an antigen binding site on the surface of the V_{H}-V_{L} dimer. Accordingly, these six CDRs give an antibody an antigen-binding specificity. The Fv can be produced by inserting a DNA encoding Fv of a LOX-1-binding antibody described herein into a prokaryotic expression vector or a eukaryotic expression vector, and by introducing the vector into a prokaryote or a eukaryote to express the DNA.

In addition, Fab is an antibody fragment having an antigen-binding activity, the fragment being obtained by treating IgG with a protease, papain, and the fragment having the N-terminal half of the H chain and the entire L chain linked via a disulfide bond. For example, the Fab can be obtained by treating a LOX-1-binding antibody described herein with a protease, papain. Alternatively, the Fab can be produced by inserting a DNA encoding Fab of the LOX-1-binding antibody into a prokaryotic expression vector or a eukaryotic expression vector, and by introducing the vector into a prokaryote or a eukaryote to express the DNA.

In addition, F(ab')₂ is an antibody fragment having an antigen-binding activity, the fragment being obtained by treating IgG with a protease, pepsin, and the fragment having a little larger portion than Fabs whose hinge regions are linked via disulfide bonds. For example, the F(ab')₂ can be obtained by treating a LOX-1-binding antibody described herein with a protease, pepsin. Also, the F(ab')₂ can be produced by linking the following Fab's via a thioether bond or a disulfide bond.

In addition, Fab' is an antibody fragment having an antigen-binding activity, the fragment being produced by cleaving the disulfide bonds in the hinge regions of the F(ab')₂. The Fab' can be yielded by treating the F(ab')₂ with a reducing agent, dithiothreitol. For example, the Fab' can be produced by inserting a DNA encoding the Fab' fragment of the LOX-1-binding antibody described herein into a prokaryotic expression vector or a eukaryotic expression vector, and by introducing the vector into a prokaryote or a eukaryote to express the DNA.

In addition, scFv is an antibody fragment having an antigen-binding activity, the fragment being a polypeptide having one V_{H} and one V_{L} linked by a suitable peptide linker. For example, the scFv can be produced by obtaining cDNAs encoding the V_{H} and V_{L} of a LOX-1-binding antibody described herein, by constructing a DNA encoding the scFv, by inserting the DNA into a prokaryotic expression vector or a eukaryotic expression vector, and by introducing the vector into a prokaryote or a eukaryote to express the DNA.

In addition, a diabody is an antibody fragment having divalent antigen-binding activities, the fragment having scFvs dimerized. Both of the divalent antigen-binding activities can be identical, or one of them can be a distinct antigen-binding activity. For example, the diabody can be produced by obtaining cDNAs encoding the V_{H} and V_{L} of a LOX-1-binding antibody described herein, by constructing a DNA encoding the scFvs with a peptide linker having a length of amino acid sequence at 8 residues or less, by inserting the DNA into a prokaryotic expression vector or a eukaryotic expression vector, and by introducing the expression vector into a prokaryote or a eukaryote to express the DNA.

In addition, dsFv is a general term referring to a polypeptide having one amino acid residue in the respective V_{H} and the V_{L} substituted by a cysteine residue, followed by linking the cysteine residues via a disulfide bond. The amino acid residue substituted by the cysteine residue can be selected based on a conformation prediction of an antibody in accordance with a procedure indicated by Reiter *et al.* [Reiter et al., Protein Eng. 1994 May, 7(5), 697-704]. For example, the dsFv can be produced by obtaining cDNAs encoding the V_{H} and V_{L} of a LOX-1-binding antibody described herein, by constructing a DNA encoding the dsFv, by inserting the DNA into a prokaryotic expression vector or a eukaryotic expression vector, and by introducing the expression vector into a prokaryote or a eukaryote to express the DNA.

In addition, a CDR-containing peptide includes at least one CDR of either the V_{H} or V_{L}. A plurality of peptides containing a CDR(s) can be linked directly or indirectly via a suitable peptide linker. For example, the peptide containing a CDR(s) can be produced by constructing a DNA encoding the V_{H} and V_{L} of a LOX-1-binding antibody described herein, by inserting the DNA into a prokaryotic expression vector or a eukaryotic expression vector, and by introducing the expression vector into a prokaryote or a eukaryote to express the DNA. In addition, the peptide containing a CDR(s) can also be produced by a chemical synthesis process such as an Fmoc process (fluorenylmethyloxycarbonyl process) and a tBOC process (t-butyloxycarbonyl process).

In addition, a chimeric antibody can be produced by linking variable regions of an antibody derived from a non-human species to a constant region of a human antibody, and can be easily constructed using gene recombinant technology. A process for producing a chimeric antibody is known in the art. For example, a mouse-human chimeric antibody can be produced by a process disclosed in Roguska et al., Proc Natl Acad Sci USA. 1994 Feb 1, 91(3), 969-973. The mouse-human chimeric antibody can be obtained by cloning DNA fragments encoding V regions of murine light and heavy chains of a murine monoclonal antibody against a target antigen, by linking the DNAs encoding these murine V regions to a DNA encoding constant regions of a human antibody, and by expressing the DNAs. A basic procedure for producing a mouse-human chimeric antibody includes the steps of: isolating a murine leader sequence and a V region sequence present in a cloned cDNA; and linking these sequences to a sequence encoding a C region of a human antibody, the sequence being present in an expression vector used for mammalian cells. Alternatively, a murine leader sequence and a V region sequence present in a cloned cDNA are first linked to a sequence encoding a C region of a human antibody and the resulting sequence is then ligated into an expression vector used for mammalian cells. A fragment of the C region of the human antibody can be a C region of an H chain or a C region of an L chain of any human antibody. Examples of the C region of the human H chain can include Cγ1, Cγ2, Cγ3 and Cγ4, and Examples of the C region of the L chain can include Cλ and Cκ. Furthermore, a chicken-human chimeric antibody can be prepared using a procedure similar to that for the above mouse-human chimeric antibody. Alternatively, the chicken-human chimeric antibody can be produced using a procedure disclosed in Nishibori et al., Biologicals 2004 Dec., 32(4), 213-218; JP2006-282521A; or JP2005-245337A.

In addition, a humanized antibody has one or more complementarity determining regions (CDRs) derived from a non-human species, human-immunoglobulin-derived framework regions (FRs), and human-immunoglobulin-derived constant regions, and the humanized antibody binds to a desired antigen. In order to modify or, preferably, improve the antigen binding, residues corresponding to those of the CDR-donor antibody are frequently substituted by amino acid residues in the human framework regions. These framework substitutions are carried out using a well-known procedure in the art (e.g., by modeling of an interaction between CDR and framework residues so as to identify a critical framework residue for the antigen binding, and by sequence comparison so as to identify an abnormal framework residue in a particular position)[Riechmann et al., Nature 1988 Mar 24, 332(6162), 323-327]. An antibody can be humanized by using various techniques known in the art [Almagro et al., FRont Biosci. 2008 Jan 1, 13, 1619-1633]. Examples of the techniques can include CDR grafting [Ozaki et al., Blood 1999 Jun 1, 93(11), 3922-3930], re-surfacing [roguska et al., Proc Natl Acad Sci USA. 1994 Feb 1, 91(3), 969-973], and FR shuffling [Damschroder et al., Mol Immunol. 2007 Apr., 44(11), 3049-3060, Epub 2007 Jan 22]. In addition, when CDRs derived from a chicken are used, a humanized antibody can be produced using a process disclosed in JP2006-241026A.

In addition, a human antibody contains a heavy chain variable region, a heavy chain constant region, a light chain variable region, and a light chain constant region, all of which constitute an antibody and are derived from genes encoding a human immunoglobulin. The human antibody has less immunogenicity at the time of administration to a human subject, and can thus preferably be used for treatment of human diseases. Examples of a basic method for the antibody production include a human-antibody-producing transgenic mouse method, a phage display technique, and the like. The human-antibody-producing transgenic mouse method includes the steps of: introducing a functional human Ig gene into an endogenous-Ig-knockout mouse; and producing, instead of a murine antibody, a human antibody having versatile antigen-binding abilities. Further, if this mouse is immunized, a human monoclonal antibody can be obtained using a conventional hybridoma method. For example, the human antibody can be prepared using a method disclosed in Lonberg et al., Int Rev Immunol. 1995, 13(1), 65-93. The phage display technique is a system in which an exogenous gene is made to be expressed at an N-terminal portion of a coat protein (e.g., g3p, g10p) of a filamentous phage such as an E. coli virus M 13 and T7, as a fusion protein without losing infectivity of the phage. For example, the human antibody can be prepared using a method disclosed in Vaughan et al., Nat Biotechnol. 1996 Mar., 14(3), 309-314.

As used herein, heavy chain CDR1, heavy chain CDR2, or heavy chain CDR3 of a LOX-1-binding antibody may be those derived from, for example, a human or other mammals (e.g., a rat, a mouse, a rabbit, a chicken, cattle, a monkey, a pig, a horse, a sheep, a goat, a dog, a cat, a guinea pig, a hamster), and may preferably be those of a chicken in particular. This is because a chicken is phylogenetically distinct from mammals, and is thus more likely to efficiently produce an antibody that cannot be generated in the mammals [Nakamura et al., J Immunol Methods 2003 Sep., 280(1-2), 157-164] .

In addition, a LOX-1-binding antibody described herein can be obtained by isolating a DNA encoding CDRs of the heavy chain of the LOX-1-binidng antibody and a DNA encoding regions other than the CDRs of the heavy chain of a known antibody derived from a human or non-human organisms, by ligating these DNAs into a vector in accordance with a known procedure in the art, and then by expressing these DNAs. At this time, in order to increase efficiency of binding of the antibody to a target antigen, it is preferable to optimize regions except the CDRs of the heavy chain of the antibody by using a known process in the art (e.g., a phage display technique or a process for screening an antibody having high reactivity by mutating, at random, amino acid residues of the antibody). In particular, in order to increase efficiency of binding of the antibody to a target antigen, FR regions are preferably optimized by using FR shuffling [Damschroder et al., Mol Immunol. 2007 Apr., 44(11), 3049-3060, Epub 2007 Jan 22] or a process for substituting packaging residues and/or amino acid residues within a vernier zone [JP2006-241026A or Foote et al., J Mol Biol. 1992 Mar 20, 224(2), 487-499].

In addition, a LOX-1-binding antibody herein can be obtained in a similar manner by isolating a DNA encoding heavy chain CDR3 of the LOX-1-binidng antibody and a DNA encoding regions other than the heavy chain CDR3 of a known antibody derived from a human or non-human organisms, by ligating these DNAs into a vector in accordance with a known procedure in the art, and then by expressing these DNAs. At this time, in order to increase efficiency of binding of the antibody to a target antigen, it is preferable to optimize regions except the heavy chain CDR3 of the antibody by using a known process in the art (e.g., a phage display technique or a process for screening an antibody having high reactivity by mutating, at random, amino acid residues of the antibody). In particular, in order to increase efficiency of binding of the antibody to a target antigen, FR regions are preferably optimized by using FR shuffling [Damschroder et al., Mol Immunol. 2007 Apr., 44(11), 3049-3060, Epub 2007 Jan 22] or a process for substituting packaging residues and/or amino acid residues within a vernier zone [JP2006-241026A or Foote et al., J Mol Biol. 1992 Mar 20, 224(2), 487-499].

Examples of a class of the LOX-1-binding antibody herein include IgM, IgD, IgG, IgA, IgE, IgX, IgY, IgW, and IgNAR. Preferably, the class is IgM, IgD, IgG, IgA, or IgE. This is because IgM, IgD, IgG, IgA, and IgE are classes possessed by a human-derived antibody. Thus, when the antibody is used as a therapeutic agent, its immunogenicity is highly likely to decrease. Furthermore, the class of the LOX-1-binding antibody is more preferably IgG4. This is because IgG4 is less susceptible to complement dependent cytotoxicity (CDC) and antibody-dependent cellular cytotoxicity (ADCC) *in vivo,* and when the antibody is administered as a therapeutic agent utilizing an effect of inhibiting antigen functions, unexpected effects other than the above effect can be avoided.

As used herein, the LOX-1-binding antibodies include a LOX-1-binding antibody that specifically binds to an N-terminal intracellular domain, a transmembrane domain, a Neck domain, or a CTL domain within a LOX-1 protein. Preferably, the LOX-1-binding antibodies include a LOX-1-binding antibody that specifically binds to the CTL domain. This is because the CTL domain is a particularly important domain for LOX-1 functions, and binding of the antibody to the domain seems to achieve an effect of inhibiting LOX-1 functions. Further, the antibody having an effect of inhibiting LOX-1 functions seems to be suitably used for applications as a therapeutic and/or diagnostic agent for diseases such as a cardiovascular disease and an atherosclerotic disease which are involved with LOX-1. Moreover, Examples below demonstrate that a LOX-1-binding antibody binding to the CTL domain has an extremely high association rate constant (ka) for LOX-1. Accordingly, it is preferable that the LOX-1-binding antibody specifically binds to the CTL domain.

As used herein, the LOX-1-binding antibodies include a LOX-1-binding antibody which binds to a wild-type or mutated LOX-1 protein. Preferably, the LOX-1 protein is a wild-type form. The mutated LOX-1 protein has preferably 30% homology to the wild-type amino acid sequence, more preferably 40% homology, still more preferably 50% homology, still more preferably 60% homology, still more preferably 70% homology, still more preferably 80% homology, still more preferably 90% homology, and still more preferably 95% homology. This is because if the mutated LOX-1 has an amino acid sequence having higher homology to the wild type LOX-1, it will have a similar function to a LOX-1-binding antibody which binds to LOX-1 having the wild-type amino acid sequence, the antibody having an extremely high association rate constant (ka) for LOX-1 as evidenced by Examples below.

### <Embodiment 2 : Production of LOX-1-binding antibody containing, as heavy chain CDR3, amino acid sequence set forth in SEQ ID NO: 4 or homologous sequence thereof>

LOX-1-binding antibodies according to this embodiment include a LOX-1-binding antibody having, as heavy chain CDR3, an amino acid sequence set forth in SEQ ID NO: 4. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, mouse, pig, and rabbit LOX-1. In view of this, the LOX-1-binding antibody seems to be suitably used for applications as a therapeutic and/or diagnostic agent for diseases such as a cardiovascular disease and an atherosclerotic disease which are involved with LOX-1.

Here, the LOX-1-binding antibodies include a LOX-1-binding antibody whose heavy chain CDR3 has 80% or more homology to the amino acid sequence set forth in SEQ ID NO: 4. In addition, the heavy chain CDR3 has preferably 85% or more homology to the amino acid sequence set forth in SEQ ID NO: 4, more preferably 90% or more, still more preferably 95% or more, and still more preferably 98% or more. This is because if the heavy chain CDR3 contains an amino acid sequence having higher homology to the sequence set forth in SEQ ID NO: 4, it will have a similar function to the LOX-1-binding antibody whose heavy chain CDR3 has the amino acid sequence set forth in SEQ ID NO: 4, the antibody having an extremely high association rate constant (ka) for LOX-1 as evidenced by Examples below.

In addition, the LOX-1-binding antibodies include a LOX-1-binding antibody whose heavy chain CDR3 has one or several amino acid deletions, substitutions, or additions in the amino acid sequence set forth in SEQ ID NO: 4. Here, the heavy chain CDR3 contains an amino acid sequence having preferably one to three amino acid deletions, substitutions, or additions in the amino acid sequence set forth in SEQ ID NO: 4, more preferably one or two amino acid deletions, substitutions, or additions, and still more preferably one amino acid deletion, substitution, or addition. This is because if the heavy chain CDR3 contains an amino acid sequence having higher homology to the sequence set forth in SEQ ID NO: 4, functions can be achieved which approach those of the LOX-1-binding antibody whose heavy chain CDR3 has the amino acid sequence set forth in SEQ ID NO: 4, the antibody having an extremely high association rate constant (ka) for LOX-1 as evidenced by Examples below.

In addition, the LOX-1-binding antibodies include a LOX-1-binding antibody whose heavy chain CDR3 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 4.

In addition, the LOX-1-binding antibodies include a LOX-1-binding antibody whose heavy chain CDR1 has the amino acid sequence set forth in SEQ ID NO: 8. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, mouse, pig, and rabbit LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR1 has 85% or more homology to the amino acid sequence set forth in SEQ ID NO: 8. Here, the heavy chain CDR1 has preferably 90% or more homology to the amino acid sequence set forth in SEQ ID NO: 8, more preferably 95% or more, and still more preferably 98% or more. This is because if the heavy chain CDR1 contains an amino acid sequence having higher homology to the sequence set forth in SEQ ID NO: 8, functions can be achieved which approach those of the LOX-1-binding antibody whose heavy chain CDR1 has the amino acid sequence set forth in SEQ ID NO: 8, the antibody having an extremely high association rate constant (ka) for LOX-1 as evidenced by Examples below.

In addition, the LOX-1-binding antibodies include a LOX-1-binding antibody whose heavy chain CDR1 has one amino acid deletion, substitution, or addition in the amino acid sequence set forth in SEQ ID NO: 8.

In addition, the LOX-1-binding antibodies include a LOX-1-binding antibody whose heavy chain CDR1 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 8.

In addition, the LOX-1-binding antibodies include a LOX-1-binding antibody whose heavy chain CDR2 has the amino acid sequence set forth in SEQ ID NO: 9. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, mouse, pig, and rabbit LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR2 has 85% or more homology to the amino acid sequence set forth in SEQ ID NO: 9. Here, the heavy chain CDR2 has preferably 90% or more homology to the amino acid sequence set forth in SEQ ID NO: 3, more preferably 95% or more, and still more preferably 98% or more. This is because if the heavy chain CDR2 contains an amino acid sequence having higher homology to the sequence set forth in SEQ ID NO: 9, functions can be achieved which approach those of the LOX-1-binding antibody whose heavy chain CDR2 has the amino acid sequence set forth in SEQ ID NO: 9, the antibody having an extremely high association rate constant (ka) for LOX-1 as evidenced by Examples below.

In addition, the LOX-1-binding antibodies include a LOX-1-binding antibody whose heavy chain CDR2 has one or several amino acid deletions, substitutions, or additions in the amino acid sequence set forth in SEQ ID NO: 9. In addition, the heavy chain CDR2 contains an amino acid sequence having preferably one to three amino acid deletions, substitutions, or additions in the amino acid sequence set forth in SEQ ID NO: 9, more preferably one or two amino acid deletions, substitutions, or additions, and still more preferably one amino acid deletion, substitution, or addition. This is because if the heavy chain CDR2 contains an amino acid sequence having higher homology to the sequence set forth in SEQ ID NO: 9, functions can be achieved which approach those of the LOX-1-binding antibody whose heavy chain CDR2 has the amino acid sequence set forth in SEQ ID NO: 9, the antibody having an extremely high association rate constant (ka) for LOX-1 as evidenced by Examples below.

In addition, the LOX-1-binding antibodies include a LOX-1-binding antibody whose heavy chain CDR2 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 9.

Also, the LOX-1-binding antibodies include a LOX-1-binding antibody which binds to LOX-1 proteins derived from mammals of different species. The mammals of different species herein refer to a human and one or more species selected from a mouse, a rat, a rabbit, a pig, a sheep, cattle, a horse, a cat, a dog, a monkey, and a chimpanzee. This is because when a therapeutic and/or diagnostic agent for a human disease is developed, the following mammals can be used as typical disease model animals, the mammals including a mouse, a rat, a rabbit, a pig, a sheep, cattle, a horse, a cat, a dog, a monkey, and a chimpanzee. In addition, the mammals are a human and preferably any of one or more species selected from a mouse, a rat, a rabbit, and a pig. This is because when a therapeutic and/or diagnostic agent for a disease such as a cardiovascular disease and a atherosclerotic disease is developed, a mouse, a rat, a rabbit, and a pig have been already generally utilized as model animals.

Furthermore, the mammals are, particularly preferably, a human, a mouse, a rabbit and a pig. This is because a LOX-1-binding antibody which binds to LOX-1 proteins derived from a human, a mouse, a rabbit, and a pig has been actually obtained in Examples below and the obtained antibody against the LOX-1 proteins has exhibited an extremely high association rate constant (ka) for LOX-1.

### <Embodiment 3 : Production of LOX-1-binding antibody containing, as heavy chain CDR3, amino acid sequence set forth in SEQ ID NO: 5 or homologous sequence thereof>

LOX-1-binding antibodies according to this embodiment include a LOX-1-binding antibody having, as heavy chain CDR3, an amino acid sequence set forth in SEQ ID NO: 5. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human and mouse LOX-1. In view of this, the LOX-1-binding antibody seems to be suitably used for applications as a therapeutic and/or diagnostic agent for diseases such as a cardiovascular disease and an atherosclerotic disease which are involved with LOX-1.

Here, the LOX-1-binding antibodies include a LOX-1-binding antibody whose heavy chain CDR3 has 75% or more homology to the amino acid sequence set forth in SEQ ID NO: 5. Here, the heavy chain CDR3 has preferably 80% or more homology to the amino acid sequence set forth in SEQ ID NO: 5, more preferably 85% or more, still more preferably 90% or more, still more preferably 95% or more, and still more preferably 98% or more. This is because if the heavy chain CDR3 contains an amino acid sequence having higher homology to the sequence set forth in SEQ ID NO: 5, functions can be achieved which approach those of the LOX-1-binding antibody whose heavy chain CDR3 has the amino acid sequence set forth in SEQ ID NO: 5, the antibody having an extremely high association rate constant (ka) for LOX-1 as evidenced by Examples below.

In addition, the LOX-1-binding antibodies include a LOX-1-binding antibody whose heavy chain CDR3 has one or several amino acid deletions, substitutions, or additions in the amino acid sequence set forth in SEQ ID NO: 5. Here, the heavy chain CDR3 contains an amino acid sequence having preferably one to three amino acid deletions, substitutions, or additions in the amino acid sequence set forth in SEQ ID NO: 5, more preferably one or two amino acid deletions, substitutions, or additions, and still more preferably one amino acid deletion, substitution, or addition. This is because if the heavy chain CDR3 contains an amino acid sequence having higher homology to the sequence set forth in SEQ ID NO: 5, functions can be achieved which approach those of the LOX-1-binding antibody whose heavy chain CDR3 has the amino acid sequence set forth in SEQ ID NO: 5, the antibody having an extremely high association rate constant (ka) for LOX-1 as evidenced by Examples below.

In addition, the LOX-1-binding antibodies include a LOX-1-binding antibody whose heavy chain CDR3 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 5.

In addition, the LOX-1-binding antibodies include a LOX-1-binding antibody whose heavy chain CDR1 has the amino acid sequence set forth in SEQ ID NO: 10. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human and mouse LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR1 has 90% or more homology to the amino acid sequence set forth in SEQ ID NO: 10. Here, the heavy chain CDR1 has preferably 95% or more homology to the amino acid sequence set forth in SEQ ID NO: 10, and more preferably 98% or more. This is because if the heavy chain CDR1 contains an amino acid sequence having higher homology to the sequence set forth in SEQ ID NO: 10, functions can be achieved which approach those of the LOX-1-binding antibody whose heavy chain CDR1 has the amino acid sequence set forth in SEQ ID NO: 10, the antibody having an extremely high association rate constant (ka) for LOX-1 as evidenced by Examples below.

In addition, the LOX-1-binding antibodies include a LOX-1-binding antibody whose heavy chain CDR1 has one amino acid deletion, substitution, or addition in the amino acid sequence set forth in SEQ ID NO: 10.

In addition, the LOX-1-binding antibodies include a LOX-1-binding antibody whose heavy chain CDR1 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 10.

In addition, the LOX-1-binding antibodies include a LOX-1-binding antibody whose heavy chain CDR2 has the amino acid sequence set forth in SEQ ID NO: 11. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human and mouse LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR2 has 85% or more homology to the amino acid sequence set forth in SEQ ID NO: 11. Here, the heavy chain CDR2 has preferably 90% or more homology to the amino acid sequence set forth in SEQ ID NO: 6, more preferably 95% or more, and still more preferably 98% or more. This is because if the heavy chain CDR2 contains an amino acid sequence having higher homology to the sequence set forth in SEQ ID NO: 11, it will have a similar function to the LOX-1-binding antibody whose heavy chain CDR2 has the amino acid sequence set forth in SEQ ID NO: 11, the antibody having an extremely high association rate constant (ka) for LOX-1 as evidenced by Examples below.

In addition, the LOX-1-binding antibodies include a LOX-1-binding antibody whose heavy chain CDR2 has one or several amino acid deletions, substitutions, or additions in the amino acid sequence set forth in SEQ ID NO: 11. Here, the heavy chain CDR2 contains an amino acid sequence having preferably one to three amino acid deletions, substitutions, or additions in the amino acid sequence set forth in SEQ ID NO: 11, more preferably one or two amino acid deletions, substitutions, or additions, and still more preferably one amino acid deletion, substitution, or addition. This is because if the heavy chain CDR2 contains an amino acid sequence having higher homology to the sequence set forth in SEQ ID NO: 11, functions can be achieved which approach those of the LOX-1-binding antibody whose heavy chain CDR2 has the amino acid sequence set forth in SEQ ID NO: 11, the antibody having an extremely high association rate constant (ka) for LOX-1 as evidenced by Examples below.

In addition, the LOX-1-binding antibodies include a LOX-1-binding antibody whose heavy chain CDR2 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 11.

In addition, the LOX-1-binding antibodies include a LOX-1-binding antibody which binds to LOX-1 proteins derived from mammals of different species. The mammals of different species herein refer to a human and one or more species selected from a mouse, a rat, a rabbit, a pig, a sheep, cattle, a horse, a cat, a dog, a monkey, and a chimpanzee. This is because when a therapeutic and/or diagnostic agent for a human disease is developed, the following mammals can be used as typical disease model animals, the mammals including a mouse, a rat, a rabbit, a pig, a sheep, cattle, a horse, a cat, a dog, a monkey, and a chimpanzee. In addition, the mammals are a human and preferably any of one or more species selected from a mouse, a rat, a rabbit, and a pig. This is because when a therapeutic and/or diagnostic agent for a disease such as a cardiovascular disease and a atherosclerotic disease is developed, a mouse, a rat, a rabbit, and a pig have been already generally utilized as model animals.

Furthermore, the mammals are, particularly preferably, a human and a mouse. This is because a LOX-1-binding antibody which binds to LOX-1 proteins derived from a human and a mouse has been actually obtained in Examples below and the obtained LOX-1-binding antibody has exhibited an extremely high association rate constant (ka) for LOX-1.

### <Embodiment 4 : Production of LOX-1-binding antibody containing, as heavy chain CDR3, amino acid sequence set forth in SEQ ID NO: 6 or homologous sequence thereof>

LOX-1-binding antibodies according to this embodiment include a LOX-1-binding antibody having, as heavy chain CDR3, an amino acid sequence set forth in SEQ ID NO: 6. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, rabbit, and pig LOX-1. In view of this, the LOX-1-binding antibody seems to be suitably used for applications as a therapeutic and/or diagnostic agent for diseases such as a cardiovascular disease and an atherosclerotic disease which are involved with LOX-1.

Here, the LOX-1-binding antibodies include a LOX-1-binding antibody whose heavy chain CDR3 has 80% or more homology to the amino acid sequence set forth in SEQ ID NO: 6. In addition, the heavy chain CDR3 has preferably 85% or more homology to the amino acid sequence set forth in SEQ ID NO: 6, more preferably 90% or more, still more preferably 95% or more, and still more preferably 98% or more. This is because if the heavy chain CDR3 contains an amino acid sequence having higher homology to the sequence set forth in SEQ ID NO: 6, it will have a similar function to the LOX-1-binding antibody whose heavy chain CDR3 has the amino acid sequence set forth in SEQ ID NO: 6, the antibody having an extremely high association rate constant (ka) for LOX-1 as evidenced by Examples below.

In addition, the LOX-1-binding antibodies include a LOX-1-binding antibody whose heavy chain CDR3 has one or several amino acid deletions, substitutions, or additions in the amino acid sequence set forth in SEQ ID NO: 6. Here, the heavy chain CDR3 contains an amino acid sequence having preferably one to three amino acid deletions, substitutions, or additions in the amino acid sequence set forth in SEQ ID NO: 6, more preferably one or two amino acid deletions, substitutions, or additions, and still more preferably one amino acid deletion, substitution, or addition. This is because if the heavy chain CDR3 contains an amino acid sequence having higher homology to the sequence set forth in SEQ ID NO: 6, it will have a similar function to the LOX-1-binding antibody whose heavy chain CDR3 has the amino acid sequence set forth in SEQ ID NO: 6, the antibody having an extremely high association rate constant (ka) for LOX-1 as evidenced by Examples below.

In addition, the LOX-1-binding antibodies include a LOX-1-binding antibody whose heavy chain CDR3 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 6.

In addition, the LOX-1-binding antibodies include a LOX-1-binding antibody whose heavy chain CDR1 has the amino acid sequence set forth in SEQ ID NO: 12. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, rabbit, and pig LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR1 has 90% or more homology to the amino acid sequence set forth in SEQ ID NO: 12. Here, the heavy chain CDR1 has preferably 95% or more homology to the amino acid sequence set forth in SEQ ID NO: 12, and more preferably 98% or more. This is because if the heavy chain CDR1 contains an amino acid sequence having higher homology to the sequence set forth in SEQ ID NO: 12, functions can be achieved which approach those of the LOX-1-binding antibody whose heavy chain CDR1 has the amino acid sequence set forth in SEQ ID NO: 12, the antibody having an extremely high association rate constant (ka) for LOX-1 as evidenced by Examples below.

In addition, the LOX1-binding antibodies include a LOX-1-binding antibody whose heavy chain CDR1 has one amino acid deletion, substitution, or addition in the amino acid sequence set forth in SEQ ID NO: 12.

In addition, the LOX-1-binding antibodies include a LOX-1-binding antibody whose heavy chain CDR1 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 12.

In addition, the LOX-1-binding antibodies include a LOX-1-binding antibody whose heavy chain CDR2 has the amino acid sequence set forth in SEQ ID NO: 13. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human, rabbit, and pig LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR2 has 85% or more homology to the amino acid sequence set forth in SEQ ID NO: 13. Here, the heavy chain CDR2 has preferably 90% or more homology to the amino acid sequence set forth in SEQ ID NO: 13, more preferably 95% or more, and still more preferably 98% or more. This is because if the heavy chain CDR2 contains an amino acid sequence having higher homology to the sequence set forth in SEQ ID NO: 13, functions can be achieved which approach those of the LOX-1-binding antibody whose heavy chain CDR2 has the amino acid sequence set forth in SEQ ID NO: 13, the antibody having an extremely high association rate constant (ka) for LOX-1 as evidenced by Examples below.

In addition, the LOX-1-binding antibodies include a LOX-1-binding antibody whose heavy chain CDR2 has one or several amino acid deletions, substitutions, or additions in the amino acid sequence set forth in SEQ ID NO: 13. Here, the heavy chain CDR2 contains an amino acid sequence having preferably one to three amino acid deletions, substitutions, or additions in the amino acid sequence set forth in SEQ ID NO: 13, more preferably one or two amino acid deletions, substitutions, or additions, and still more preferably one amino acid deletion, substitution, or addition. This is because if the heavy chain CDR2 contains an amino acid sequence having higher homology to the sequence set forth in SEQ ID NO: 13, functions can be achieved which approach those of the LOX-1-binding antibody whose heavy chain CDR2 has the amino acid sequence set forth in SEQ ID NO: 13, the antibody having an extremely high association rate constant (ka) for LOX-1 as evidenced by Examples below.

In addition, the LOX-1-binding antibodies include a LOX-1-binding antibody whose heavy chain CDR2 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 13.

Also, the LOX-1-binding antibodies include a LOX-1-binding antibody which binds to LOX-1 proteins derived from mammals of different species. The mammals of different species herein refer to a human and one or more species selected from a mouse, a rat, a rabbit, a pig, a sheep, cattle, a horse, a cat, a dog, a monkey, and a chimpanzee. This is because when a therapeutic and/or diagnostic agent for a human disease is developed, the following mammals can be used as typical disease model animals, the mammals including a mouse, a rat, a rabbit, a pig, a sheep, cattle, a horse, a cat, a dog, a monkey, and a chimpanzee. In addition, the mammals are a human and preferably any of one or more species selected from a mouse, a rat, a rabbit, and a pig. This is because when a therapeutic and/or diagnostic agent for a disease such as a cardiovascular disease and a atherosclerotic disease is developed, a mouse, a rat, a rabbit, and a pig have been already generally utilized as model animals.

Furthermore, the mammals are, particularly preferably, a human, a rabbit and a pig. This is because a LOX-1-binding antibody which binds to LOX-1 proteins derived from a human, a rabbit, and a pig has been actually obtained in Examples below and the obtained LOX-1-binding antibody has exhibited an extremely high association rate constant (ka) for LOX-1.

### <Embodiment 5 : Production of LOX-1-binding antibody containing, as heavy chain CDR3, amino acid sequence set forth in SEQ ID NO: 7 or homologous sequence thereof>

LOX-1-binding antibodies according to this embodiment include a LOX-1-binding antibody having, as heavy chain CDR3, an amino acid sequence set forth in SEQ ID NO: 7. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human and pig LOX-1. In view of this, the LOX-1-binding antibody seems to be suitably used for applications as a therapeutic and/or diagnostic agent for diseases such as a cardiovascular disease and an atherosclerotic disease which are involved with LOX-1.

Here, the LOX-1-binding antibodies include a LOX-1-binding antibody whose heavy chain CDR3 has 80% or more homology to the amino acid sequence set forth in SEQ ID NO: 7. In addition, the heavy chain CDR3 has preferably 85% or more homology to the amino acid sequence set forth in SEQ ID NO: 7, more preferably 90% or more, still more preferably 95% or more, and still more preferably 98% or more. This is because if the heavy chain CDR3 contains an amino acid sequence having higher homology to the sequence set forth in SEQ ID NO: 7, it will have a similar function to the LOX-1-binding antibody whose heavy chain CDR3 has the amino acid sequence set forth in SEQ ID NO: 7, the antibody having an extremely high association rate constant (ka) for LOX-1 as evidenced by Examples below.

In addition, the LOX-1-binding antibodies include a LOX-1-binding antibody whose heavy chain CDR3 has one or several amino acid deletions, substitutions, or additions in the amino acid sequence set forth in SEQ ID NO: 7. Here, the heavy chain CDR3 contains an amino acid sequence having preferably one to three amino acid deletions, substitutions, or additions in the amino acid sequence set forth in SEQ ID NO: 7, more preferably one or two amino acid deletions, substitutions, or additions, and still more preferably one amino acid deletion, substitution, or addition. This is because if the heavy chain CDR3 contains an amino acid sequence having higher homology to the sequence set forth in SEQ ID NO: 7, it will have a similar function to the LOX-1-binding antibody whose heavy chain CDR3 has the amino acid sequence set forth in SEQ ID NO: 7, the antibody having an extremely high association rate constant (ka) for LOX-1 as evidenced by Examples below.

In addition, the LOX-1-binding antibodies include a LOX-1-binding antibody whose heavy chain CDR3 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 7.

In addition, the LOX-1-binding antibodies include a LOX-1-binding antibody whose heavy chain CDR1 has the amino acid sequence set forth in SEQ ID NO: 14. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human and pig LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR1 has 90% or more homology to the amino acid sequence set forth in SEQ ID NO: 14. Here, the heavy chain CDR1 has preferably 95% or more homology to the amino acid sequence set forth in SEQ ID NO: 14, and more preferably 98% or more. This is because if the heavy chain CDR1 contains an amino acid sequence having higher homology to the sequence set forth in SEQ ID NO: 14, it will have a similar function to the LOX-1-binding antibody whose heavy chain CDR1 has the amino acid sequence set forth in SEQ ID NO: 14, the antibody having an extremely high association rate constant (ka) for LOX-1 as evidenced by Examples below.

In addition, the LOX-1-binding antibodies include a LOX-1-binding antibody whose heavy chain CDR1 has one amino acid deletion, substitution, or addition in the amino acid sequence set forth in SEQ ID NO: 14.

In addition, the LOX-1-binding antibodies include a LOX-1-binding antibody whose heavy chain CDR1 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 14.

In addition, the LOX-1-binding antibodies include a LOX-1-binding antibody whose heavy chain CDR2 has the amino acid sequence set forth in SEQ ID NO: 15. At this occasion, it has been demonstrated in Examples below that the LOX-1-binding antibody exhibits an extremely high association rate constant (ka) for LOX-1. In addition, the LOX-1-binding antibody has been demonstrated in Examples below to inhibit the binding of LOX-1 to oxLDL. Accordingly, the LOX-1-binding antibody seems to be suitably used for applications as a therapeutic and/or diagnostic agent for diseases such as a cardiovascular disease and an atherosclerotic disease which are involved with LOX-1. Further, Examples below demonstrate that this antibody exhibits cross-reactivity to human and pig LOX-1. In view of this, the LOX-1-binding antibody seems to be suitably used for applications as a therapeutic and/or diagnostic agent for diseases such as a cardiovascular disease and an atherosclerotic disease which are involved with LOX-1.

In addition, the LOX-1-binding antibodies include a LOX-1-binding antibody whose heavy chain CDR2 has 85% or more homology to the amino acid sequence set forth in SEQ ID NO: 15. Here, the heavy chain CDR2 has preferably 90% or more homology to the amino acid sequence set forth in SEQ ID NO: 15, more preferably 95% or more, and still more preferably 98% or more. This is because if the heavy chain CDR2 contains an amino acid sequence having higher homology to the sequence set forth in SEQ ID NO: 15, functions can be achieved which approach those of the LOX-1-binding antibody whose heavy chain CDR2 has the amino acid sequence set forth in SEQ ID NO: 15, the antibody having an extremely high association rate constant (ka) for LOX-1 as evidenced by Examples below.

In addition, the LOX-1-binding antibodies include a LOX-1-binding antibody whose heavy chain CDR2 has one or several amino acid deletions, substitutions, or additions in the amino acid sequence set forth in SEQ ID NO: 15. Here, the heavy chain CDR2 contains an amino acid sequence having preferably one to three amino acid deletions, substitutions, or additions in the amino acid sequence set forth in SEQ ID NO: 15, more preferably one or two amino acid deletions, substitutions, or additions, and still more preferably one amino acid deletion, substitution, or addition. This is because if the heavy chain CDR2 contains an amino acid sequence having higher homology to the sequence set forth in SEQ ID NO: 15, it will have a similar function to the LOX-1-binding antibody whose heavy chain CDR2 has the amino acid sequence set forth in SEQ ID NO: 15, the antibody having an extremely high association rate constant (ka) for LOX-1 as evidenced by Examples below.

In addition, the LOX-1-binding antibodies include a LOX-1-binding antibody whose heavy chain CDR2 has an amino acid sequence encoded by a nucleotide sequence of a nucleic acid chain hybridizing under a stringent condition with a nucleic acid chain having a nucleotide sequence complementary to a nucleotide sequence encoding the amino acid sequence set forth in SEQ ID NO: 15.

Also, the LOX-1-binding antibodies include a LOX-1-binding antibody which binds to LOX-1 proteins derived from mammals of different species. The mammals of different species herein refer to a human and one or more species selected from a mouse, a rat, a rabbit, a pig, a sheep, cattle, a horse, a cat, a dog, a monkey, and a chimpanzee. This is because when a therapeutic and/or diagnostic agent for a human disease is developed, the following mammals can be used as typical disease model animals, the mammals including a mouse, a rat, a rabbit, a pig, a sheep, cattle, a horse, a cat, a dog, a monkey, and a chimpanzee. In addition, the mammals are a human and preferably any of one or more species selected from a mouse, a rat, a rabbit, and a pig. This is because when a therapeutic and/or diagnostic agent for a disease such as a cardiovascular disease and a atherosclerotic disease is developed, a mouse, a rat, a rabbit, and a pig have been already generally utilized as model animals.

Furthermore, the mammals are, particularly preferably, a human and a pig. This is because a LOX-1-binding antibody which binds to LOX-1 proteins derived from a human and a pig has been actually obtained in Examples below and the obtained LOX-1-binding antibody has exhibited an extremely high association rate constant (ka) for LOX-1.

### <Embodiment 6: Effects of LOX-1-binding antibodies>

LOX-1-binding antibodies according to this embodiment include an inhibitor of binding of LOX-1 to oxLDL. Inhibition of the binding of LOX-1 to oxLDL seems to be effective in prevention or treatment of diseases such as a cardiovascular disease, an atherosclerotic disease, thrombosis, thrombocytopenia, a systemic inflammatory response syndrome, an inflammation, and intimal hyperplasia which are involved with LOX-1. This is because a function of inhibiting the binding of LOX-1 to oxLDL by the anti-LOX-1 mAbs has been demonstrated to have a therapeutic effect on a cardiovascular disease, an atherosclerotic disease, thrombosis, thrombocytopenia, a systemic inflammatory response syndrome, an inflammation, or intimal hyperplasia of mammals, which is reported in WO01/064862; Inoue et al., Circ Res. 2005 Jul 22, 97(2), 176-184; Hinagata et al., Cardiovasc Res. 2006 Jan, 69(1), 263-271; and Honjo et al., Proc Natl Acad Sci USA. 2003 Feb 4, 100(3), 1274-1279.

Here, the effect of inhibiting the binding can be measured by a known method in the art such as an ELISA, FACS analysis, and a method using BIACORE. The competitive inhibition of the binding between LOX-1 and oxLDL under the presence of both molecules by using the LOX-1-binding antibody may be measured. Alternatively, the mode of binding of LOX-1 to the LOX-1-binding antibody may be determined as an index for inhibition of binding between LOX-1 and oxLDL. In addition, the determination of an effect of inhibiting the binding is preferably measured by a method described in Examples below.

The mode of the binding of the LOX-1-binding antibody to LOX-1 can be represented by a dissociation constant (KD), an association constant (Ka), an association rate constant (ka), and/or a dissociation rate constant (kd). In general, performance of an antibody is evaluated by a dissociation constant (KD) or an association constant (Ka) in some cases. However, these constants are static parameters in which equilibrium is presumed to have been reached. In actual practice, since the reaction time is restricted, the reaction does not reach equilibrium in most cases. Accordingly, the antigen-antibody reaction should be preferably estimated in the actual practice by using a dynamic parameter of an association rate constant (ka) or a dissociation rate constant (kd). An ELISA (Enzyme Linked Immuno-Sorbent Assay) or a BIACORE system can be used for the measurement. The ELISA can be implemented with a relatively low cost, and is the most common technique. The ELISA is an assay for determining a specific interaction, including the steps of: immobilizing, on a microplate, a predetermined amount of an antigen or antibody specifically reacting with a substance of measurement subject; adding the substance of measurement subject and an enzymatically labeled antigen together to react them; and measuring an enzymatic activity of the enzymatically labeled antigen bound to the microplate by using a colorimetric method or a fluorescence method. The assay utilizes a high binding capability and molecule-recognition capability of an antibody, so that detection can be achieved with very high sensitivity, compared to a method using HPLC, etc.

The BIACORE system is an excellent measurement method which can determine a dynamic parameter. The method includes the steps of: immobilizing a biomolecule on a sensor surface; applying an interaction partner molecule; and carrying out a real-time measurement of a specific interaction on the sensor surface. Without the need for labeling molecules, the BIACORE system can measure in real-time a specific interaction from an association reaction to an equilibrium state and a dissociation reaction. Measurement manipulations include the steps of: immobilizing a ligand on a sensor surface, and thereafter; applying a sample solution containing a reaction substance into a microchannel system; and measuring a specific interaction occurring on the sensor surface as a small mass change. The measurement principle employs an optical phenomenon, what is called surface plasmon resonance (SPR), so that a reliable measurement can be carried out. An association rate constant (ka) and a dissociation rate constant (kd) can be calculated based on reaction rates directly obtained, which allows for detailed analysis [Jonsson et al., Biotechniques 1991 Nov., 11(5), 620-7; Fivash et al., Curr Opin Biotechnol. 1998 Feb., 9(1), 97-101; and "Experiment Handbook of Instrumental Analysis for Life Science", YODOSHA CO., LTD. (2007), 243-248].

The LOX-1-binding antibodies according to this embodiment include an inhibitor for uptake of oxLDL into LOX-1-expressing cells. Inhibition of the uptake of oxLDL into LOX-1-expressing cells seems to be effective in prevention or treatment of diseases such as a cardiovascular disease, an atherosclerotic disease, thrombosis, thrombocytopenia, a systemic inflammatory response syndrome, an inflammation, and intimal hyperplasia which are involved with LOX-1. This is because a function of inhibiting the uptake of oxLDL into LOX-1-expressing cells has been demonstrated to have a therapeutic effect on a cardiovascular disease, an atherosclerotic disease, thrombosis, thrombocytopenia, a systemic inflammatory response syndrome, an inflammation, or intimal hyperplasia of mammals, which is reported in WO01/064862; Inoue et al., Circ Res. 2005 Jul 22, 97(2), 176-184; Hinagata et al., Cardiovasc Res. 2006 Jan., 69(1), 263-271; and Honjo et al., Proc Natl Acad Sci USA. 2003 Feb 4, 100(3), 1274-1279.

The LOX-1-binding antibody according to this embodiment is included in a therapeutic agent for a cardiovascular disease, an atherosclerotic disease, thrombosis, thrombocytopenia, a systemic inflammatory response syndrome, an inflammation, or intimal hyperplasia of mammals. Anti-LOX-1 mAbs have been demonstrated in an *in vivo* experiment to have a therapeutic effect on a cardiovascular disease, an atherosclerotic disease, thrombosis, thrombocytopenia, a systemic inflammatory response syndrome, an inflammation, or intimal hyperplasia of mammals, which is reported in WO01/064862; Inoue et al., Circ Res. 2005 Jul 22, 97(2), 176-184; Hinagata et al., Cardiovasc Res. 2006 Jan., 69(1), 263-271; and Honjo et al., Proc Natl Acad Sci USA. 2003 Feb 4, 100(3), 1274-1279. Accordingly, the LOX-1-binding antibody according to this embodiment is also considered to exert an effect on similar diseases.

The term "treatment" or "therapy" herein refers to being capable of exerting an effect of preventing or improving a disease or one of symptoms which are accompanied by the disease.

In addition, when the LOX-1-binding antibody is used to treat or to prevent a disease, the antibody can be solely administered. However, the antibody is usually preferably provided as a pharmaceutical composition prepared using any known method in the art of pharmacy by mixing one or more pharmaceutically acceptable carriers together with the antibody. In addition, when the LOX-1-binding antibody is not directly administered, a polynucleotide encoding the LOX-1-binding antibody or a vector having the polynucleotide can be administered.

Also, when the LOX-1-binding antibody is administered *in vivo,* the most effective administration routes during treatment are preferably used. Examples of the administration route can include oral administration or parenteral administration such as intraoral, tracheobronchial, intrarectal, subcutaneous, intramuscular, intraocular, and intravenous administration. Systemic or topical administration can be allowed. The administration route can preferably include intravenous administration. When the LOX-1-binding antibody can exert a desired function in an affected area after oral administration, the oral administration is preferable.

Examples of a dosage form can include sprays, capsules, tablets, granules, syrups, emulsions, suppositories, injections, ointments, tapes, and the like. Examples of the formulation suitable for oral administration can include emulsions, syrups, capsules, tablets, powder medicines, granules, and the like. Liquid preparations such as emulsions and syrups can be prepared using additives including water, sugars (e.g., sucrose, sorbitol, fructose), glycols (e.g., polyethylene glycol, propylene glycol), oils (e.g., a sesame oil, an olive oil, a soy oil) , preservatives (e.g., p-hydroxy benzoate esters), flavors (e.g., a strawberry flavor, peppermint), and the like. Further, the capsules, tablets, powder medicines, and granules can be prepared using additives including excipients (e.g., lactose, glucose, sucrose, mannitol), disintegrants (e.g., starch, sodium alginate), lubricants (e.g., magnesium stearate, talc), binders (e.g., polyvinyl alcohol, hydroxypropylcellulose, gelatin), surfactants (e.g., a fatty acid ester), plasticizers (e.g., glycerol), and the like.

Examples of the formulation suitable for parenteral administration can include injections, suppositories, sprays, and the like. Examples of an aqueous solution used for injections can include a saline and an isotonic solution containing glucose or another adjuvant such as D-sorbitol, D-mannose, D-mannitol, and sodium chloride. The adjuvant can be combined with a solubilization aid (e.g., alcohol (e.g., ethanol)), polyalcohol (e.g., propylene glycol, polyethylene glycol), and/or a non-ionic surfactant (e.g., polysorbate 80 (TM), HCO-50). The suppositories may be prepared using a carrier such as cacao butter, hydrogenated fat, or carboxylic acid. In addition, the sprays may be prepared using the LOX-1-binding antibody and a carrier, etc., which does not stimulate an oral cavity and respiratory tract mucosa of recipients and which makes the LOX-1-binding antibody disperse as fine particles, so that the antibody is absorbed easily. Specific examples of this carrier include lactose, glycerol, and the like. Formulations such as aerosol and dry powder are allowed depending on characteristics of the LOX-1-binding antibody and the carrier used. In addition, the components exemplified as additives for oral agents can be added to even these parenteral agents.

Also, the above prophylactic or therapeutic agent may be formulated with buffers (e.g., a phosphate buffer, a sodium acetate buffer), soothing agents (e.g., benzalkonium chloride, procaine hydrochloride), stabilizers (e.g., human serum albumin, polyethylene glycol), preservatives (e.g., benzyl alcohol, phenol), antioxidants, and/or the like. Prepared injections are usually filled in suitable ampules. Formulations as obtained in such a manner are safe and less toxic. Accordingly, the formulations can be administered to a human or mammals (e.g., a rat, a mouse, a rabbit, a sheep, a pig, cattle, a cat, a dog, a monkey).

In addition, an administration procedure can be appropriately selected depending on an age, a symptom, and/or an affected organ, etc., of a patient. The dose per administration of a pharmaceutical composition containing the LOX-1-binding antibody or a polynucleotide encoding the LOX-1-binding antibody can be selected from, for example, a range between 0.0001 mg and 1000 mg per kg body weight. Alternatively, the dose can be selected from, but is not necessarily limited to, a range between 0.001 and 100000 mg per patient body. The dose varies depending on an intended therapeutic effect, an administration procedure, a treatment period, an age, and/or a body weight, etc. The dose and administration procedure vary depending on a body weight, an age, and/or a symptom, etc. of a patient. However, those skilled in the art can appropriately select them. In addition, the administration may be combined with a suitable chemotherapeutic agent.

Cardiovascular diseases (CVDs) refers to heart- and blood-vessel-system diseases including myocardial infarction, angina (including unstable angina), a valvular heart disease, aortic aneurysm, arterial rupture, arteriosclerosis obliterans, acute myocardial infarction, and the like. In addition, the cardiovascular diseases include a coronary heart disease, a cerebrovascular disease, hypertension (raised blood pressure), a peripheral artery disease, a rheumatic heart disease, a congenital heart disease, and heart failure. According to WHO (World Health Organization), the cardiovascular disease is the most frequent cause of death in the world. It has been reported that 17.5 million people died from the cardiovascular disease, which accounts for 30% of the death causes all over the world in the 2005 statics ["Cardiovascular diseases" Fact sheet N° 317 February 2007, World Health Organization].

An atherosclerotic disease refers to a disease in which artery walls thicken and primary functions of blood vessels deteriorate. Arteriosclerosis includes atherosclerosis, arteriolosclerosis, and medial sclerosis. A prophylactic or therapeutic agent for arteriosclerosis is particularly useful for atherosclerosis, the agent using the LOX-1-binding antibody as described herein. Examples of the atherosclerotic disease include cerebral infarction, intracranial hemorrhage, an ischemic heart disease (e.g., myocardial infarction, angina), aortic aneurysm, aortic dissection, nephrosclerosis or renal failure caused thereby, arteriosclerosis obliterans, and the like.

The atherosclerosis is caused by accumulation of fatty and fibrous deposition, what is known as a plaque, on an arterial wall. The plaque narrows an artery, which decreases blood supply to organs such as a heart and a brain involving life-threatening conditions. As a result, this may cause angina (i.e., a transient ischemic attack). The plaque may rapture and a blood clot may appear, which results in complete and sudden blockage of blood stream in some cases. In the heart, this blockage induces a heart attack. In the brain, a cerebral stroke occurs [Bonow et al., Circulation 2002 Sep 24, 106(13), 1602-1605].

In addition, viscosity become elevated in blood having a high lipid content, and the lipid is likely to be attached to an inner wall of an artery. Next, cholesterol invades into intima from endothelial cells which have been damaged (stimulated) by hypertension, diabetes, smoking, and stresses, etc. The cholesterol is then oxidized and denatured to become oxLDL which more readily causes arteriosclerosis. In addition, monocytes are attached to the damaged endothelial cells, invaded into intima, and then transformed into macrophages. These macrophages incorporate oxLDL and become foam cells, which leads to occurrence of intimal hyperplasia. This occurrence deteriorates blood flow and induces arteriosclerosis which is responsible for myocardial infarction, angina, and cerebral infarction, etc.

The thrombosis refers to a lesion in a heart or a portion nurtured by blood vessels, etc., in which blood is coagulated and blood flow is then blocked due to blood congestion and changes in blood vessel walls. This is often caused by arteriosclerosis, which leads to an extremely critical disease such as brain thrombosis and myocardial infarction. The term "thrombosis" includes various diseases resulting from blood coagulation in a blood vessel. Examples of the thrombosis also include cerebral infarction, myocardial infarction, deep vein thrombosis (e.g., an economy-class syndrome), unstable angina, cerebral stroke, and pulmonary embolism.

Thrombocytopenia refers to a symptom having a decreased number of platelets. Examples of the thrombocytopenia include idiopathic thrombocytopenic purpura, thrombotic thrombocytopenic purpura, and a hemolytic-uremic syndrome.

A systemic inflammatory response syndrome refers to a systemic inflammatory response triggered by various infectious diseases, severe trauma, or burn. Examples of the systemic inflammatory response syndrome include sepsis, trauma, burn, acute pancreatitis, and the like. A suitable example is sepsis. The term "sepsis" generally means a systemic inflammatory response syndrome caused by infectious diseases.

An inflammation refers to a defense response evoked by a living tissue responding to a local injury of the body, the response exhibiting symptoms such as redness, swelling, burning sensation, pain, and impairment. Examples of a disease accompanied by an inflammation include an infectious disease, rheumatoid arthritis, a connective tissue disease, gout, an allergic disease, an inflammatory bowel disease, interstitial pneumonia, and the like.

The LOX-1-binding antibody according to this embodiment is included in a diagnostic agent for a cardiovascular disease, an atherosclerotic disease, thrombosis, thrombocytopenia, a systemic inflammatory response syndrome, an inflammation, or intimal hyperplasia of mammals. Anti-LOX-1 mAbs have been demonstrated in an *in vivo* experiment to have a therapeutic effect on a cardiovascular disease, an atherosclerotic disease, thrombosis, thrombocytopenia, a systemic inflammatory response syndrome, an inflammation, or intimal hyperplasia of mammals, which is reported in WO01/064862; Inoue et al., Circ Res. 2005 Jul 22, 97(2), 176-184; Hinagata et al., Cardiovasc Res. 2006 Jan., 69(1), 263-271; and Honjo et al., Proc Natl Acad Sci USA. 2003 Feb 4, 100(3), 1274-1279. The above diseases can be diagnosed by measuring and comparing the levels of the LOX-1 expression in, for example, a standard cell, a test cell, a test serum, or a pathologic cell of any of the above diseases in accordance with the binding mode between the anti-LOX-1 mAbs and LOX-1. According to Hayashida et al., Circulation 2005 Aug 9; 112(6): 812-8, Epub 2005 Aug 1, a soluble form of LOX-1 (sLOX-1) released from serum derived from a patient with an acute coronary syndrome (ACS) by protein cleavage has been detected. In this case, serum sLOX-1 levels are determined using the anti-LOX-1 mAbs, which allows for diagnosis of the ACS.

Examples of a detection method at the time of using the mAbs as a diagnostic agent can include, but are not limited to, a radioimmunoassay, an enzyme immunoassay, a fluoroimmunoassay, a luminescence immunoassay, immunoprecipitation, immune nephelometry, and the like. The enzyme immunoassay is preferable. Particularly preferred is an ELISA (enzyme-linked immunosorbent assay)(e.g., a sandwich ELISA). The above immunological method such as an ELISA can be carried out using a procedure known to those skilled in the art.

For example, a typical detection method using the LOX-1-binding antibody can include the steps of: immobilizing the LOX-1-binding antibody on a support; adding a sample thereto; incubating them; causing the LOX-1-binding antibody to bind to a LOX-1 protein in the sample, and thereafter; washing them; and detecting the LOX-1 protein bound to the support via the LOX-1-binding antibody, thereby detecting the LOX-1 protein in the sample.

Examples of a preferable embodiment of detection of a LOX-1 protein bound to a support via the LOX-1-binding antibody can include a method using the LOX-1-binding antibody labeled with a labeled substance. For example, a test sample is made to contact the LOX-1-binding antibody immobilized on a support. After washing, a labeled-substance-labeled LOX-1-binding antibody is made to contact the test sample. Then, the labeled substance is detected using another labeled antibody to create an index for LOX-1.

The labeling of the LOX-1-binding antibody can be performed using a commonly known procedure. Examples of the labeled substance which can be used include labeled substances known to those skilled in the art, such as fluorescent dye, an enzyme, a coenzyme, a chemiluminescent substance, and a radioactive material. Specific examples of the labeled substance can include a radioisotope (e.g., ³²P, ¹⁴C, ¹²⁵I, ³H, ¹³¹I), fluorescein, rhodamine, dansyl chloride, umbelliferone, luciferase, peroxidase, alkaline phosphatase, β-galactosidase, β-glucosidase, horseradish peroxidase, glucoamylase, lysozyme, saccharide oxidase, microperoxidase, biotin, and the like. When the biotin is used as a labeled substance, a biotin-labeled antibody is added, and avidin conjugating an enzyme such as alkaline phosphatase is then further added.

Hereinafter, effects according to embodiments 1 to 6 are described.

Embodiments of the present invention provide LOX-1-binding antibodies which specifically bind to a LOX-1 protein, the antibodies having heavy chain CDR3 comprising one or more amino acid sequences selected from the group consisting of a) an amino acid sequence set forth in SEQ ID NO: 1, b) an amino acid sequence having 80% or more homology to the amino acid sequence a), c) an amino acid sequence having one or several amino acid deletions, substitutions, or additions in the amino acid sequence a), and d) an amino acid sequence encoded by a nucleotide sequence of a nucleic acid chain hybridizing under a stringent condition with a nucleic acid chain having a nucleotide sequence complementary to a nucleotide sequence encoding the amino acid sequence a). These embodiments produce effects of generating an inhibitor for binding of LOX-1 to oxLDL, an inhibitor for uptake of oxLDL into LOX-1-expressing cells, or a therapeutic or diagnostic agent for a cardiovascular disease, an atherosclerotic disease, thrombosis, thrombocytopenia, a systemic inflammatory response syndrome, an inflammation, or intimal hyperplasia of mammals.

Other embodiments of the present invention provide LOX-1-binding antibodies having: in addition to the above heavy chain CDR3, heavy chain CDR comprising one or more amino acid sequences selected from the group consisting of e) an amino acid sequence set forth in SEQ ID NO: 2, f) an amino acid sequence having 90% or more homology to the amino acid sequence e), g) an amino acid sequence having one amino acid deletion, substitution, or addition in the amino acid sequence e), and h) an amino acid sequence encoded by a nucleotide sequence of a nucleic acid chain hybridizing under a stringent condition with a nucleic acid chain having a nucleotide sequence complementary to a nucleotide sequence encoding the amino acid sequence e); and heavy chain CDR2 comprising one or more amino acid sequences selected from the group consisting of i) an amino acid sequence set forth in SEQ ID NO: 3, j) an amino acid sequence having 85% or more homology to the amino acid sequence i), k) an amino acid sequence having one or several amino acid deletions, substitutions, or additions in the amino acid sequence i), and 1) an amino acid sequence encoded by a nucleotide sequence of a nucleic acid chain hybridizing under a stringent condition with a nucleic acid chain having a nucleotide sequence complementary to a nucleotide sequence encoding the amino acid sequence i). These embodiments produce effects of generating an inhibitor for binding of LOX-1 to oxLDL or an inhibitor for uptake of oxLDL into LOX-1-expressing cells. In addition, these embodiments produce effects of generating a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a LOX-1-binding antibody which binds to LOX-1 proteins derived from mammals of different species. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a LOX-1-binding antibody which binds to any of LOX-1 proteins derived from a human, a rabbit, and a pig. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of a human, a rabbit, and a pig.

In another embodiment of the present invention, the LOX-1-binding antibody represents one or more antibodies selected from the group consisting of a chicken antibody, a chicken-human chimeric antibody, and a humanized antibody. According to this embodiment, the chicken-human chimeric antibody or the humanized antibody is particularly useful because these antibodies exert effects of decreasing immunogenicity in a human.

In another embodiment of the present invention, the LOX-1-binging antibody represents the above chicken-human chimeric antibody or the above humanized antibody whose subclass is IgG4. This embodiment is useful because IgG4 is less susceptible to complement dependent cytotoxicity (CDC) and antibody-dependent cellular cytotoxicity (ADCC) *in vivo,* and when the antibody is administered as a therapeutic agent utilizing an effect of inhibiting LOX-1 functions, unexpected effects other than the above effect can be avoided.

Another embodiment of the present invention provides a LOX-1-binding antibody which specifically binds to the CTL domain of a LOX-1 protein. This embodiment produces effects of effectively inhibiting LOX-1 functions because the antibody interacts with the CTL domain which is critical in the LOX-1 functions.

In another embodiment of the present invention, the LOX-1-binding antibody binds to a wild-type or mutant form of a LOX-1 protein. According to this embodiment, when the LOX-1 protein is mutated, there is an effect of the antibody capable of binding to the mutated protein.

In another embodiment of the present invention, the LOX-1-binding antibody is an antibody fragment. According to this embodiment, the antibody is shorter than the full-length antibody, thereby producing effects that the immunogenicity can be decreased and/or the stability of the antibody can be increased at the time of administration to a living body.

In another embodiment of the present invention, the LOX-1-binding antibody is an antibody which neutralizes LOX-1. This embodiment produces effects of generating an inhibitor for binding of LOX-1 to oxLDL or an inhibitor for uptake of oxLDL into LOX-1-expressing cells. In addition, this embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of mammals.

In another embodiment of the present invention, the LOX-1-binding antibody is a monoclonal antibody. According to this embodiment, the antibody exerts an effect of recognizing a LOX-1 protein with high specificity.

Another embodiment of the present invention provides a polynucleotide comprising a nucleotide sequence encoding the LOX-1-binding antibody. According to this embodiment, the LOX-1-binding antibody can be produced using a known method in the art.

Another embodiment of the present invention provides a vector having a polynucleotide encoding the LOX-1-binding antibody or a portion thereof. According to this embodiment, the LOX-1-binding antibody can be produced using a known method in the art.

Another embodiment of the present invention provides an inhibitor for binding of LOX-1 to oxLDL, the inhibitor containing the LOX-1-binding antibody. This embodiment produces an effect of generating an inhibitor for uptake of oxLDL into LOX-1-expressing cells. In addition, this embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides an inhibitor for uptake of oxLDL into LOX-1-expressing cells, the inhibitor containing the LOX-1-binding antibody. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a therapeutic agent for a cardiovascular disease, an atherosclerotic disease, thrombosis, thrombocytopenia, a systemic inflammatory response syndrome, an inflammation, or intimal hyperplasia of mammals. The therapeutic agent contains the LOX-1-binding antibody. According to this embodiment, an effect of treating the above diseases can be achieved by administering the antibody to a living body.

Another embodiment of the present invention provides a diagnostic agent for a cardiovascular disease, an atherosclerotic disease, thrombosis, thrombocytopenia, a systemic inflammatory response syndrome, an inflammation, or intimal hyperplasia of mammals. The diagnostic agent contains the LOX-1-binding antibody. According to this embodiment, an effect of diagnosing the above diseases can be obtained using a known method in the art.

Other embodiments of the present invention provide LOX-1-binding antibodies which specifically bind to a LOX-1 protein, the antibodies having heavy chain CDR3 comprising one or more amino acid sequences selected from the group consisting of m) an amino acid sequence set forth in SEQ ID NO: 4, n) an amino acid sequence having 80% or more homology to the amino acid sequence m), o) an amino acid sequence having one or several amino acid deletions, substitutions, or additions in the amino acid sequence m), and p) an amino acid sequence encoded by a nucleotide sequence of a nucleic acid chain hybridizing under a stringent condition with a nucleic acid chain having a nucleotide sequence complementary to a nucleotide sequence encoding the amino acid sequence m). These embodiments produce effects of generating an inhibitor for binding of LOX-1 to oxLDL, an inhibitor for uptake of oxLDL into LOX-1-expressing cells, or a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a LOX-1-binding antibody which binds to any of LOX-1 proteins derived from a human, a mouse, a rabbit, and a pig. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of a human, a mouse, a rabbit, and a pig.

Other embodiments of the present invention provide LOX-1-binding antibodies which specifically bind to a LOX-1 protein, the antibodies having heavy chain CDR3 comprising one or more amino acid sequences selected from the group consisting of q) an amino acid sequence set forth in SEQ ID NO: 5, r) an amino acid sequence having 80% or more homology to the amino acid sequence q), s) an amino acid sequence having one or several amino acid deletions, substitutions, or additions in the amino acid sequence q), and t) an amino acid sequence encoded by a nucleotide sequence of a nucleic acid chain hybridizing under a stringent condition with a nucleic acid chain having a nucleotide sequence complementary to a nucleotide sequence encoding the amino acid sequence q). These embodiments produce effects of generating an inhibitor for binding of LOX-1 to oxLDL, an inhibitor for uptake of oxLDL into LOX-1-expressing cells, or a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a LOX-1-binding antibody which binds to any of LOX-1 proteins derived from a human and a mouse. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of a human and a mouse.

Other embodiments of the present invention provide LOX-1-binding antibodies which specifically bind to a LOX-1 protein, the antibodies having heavy chain CDR3 comprising one or more amino acid sequences selected from the group consisting of u) an amino acid sequence set forth in SEQ ID NO: 6, v) an amino acid sequence having 80% or more homology to the amino acid sequence u), w) an amino acid sequence having one or several amino acid deletions, substitutions, or additions in the amino acid sequence u), and x) an amino acid sequence encoded by a nucleotide sequence of a nucleic acid chain hybridizing under a stringent condition with a nucleic acid chain having a nucleotide sequence complementary to a nucleotide sequence encoding the amino acid sequence u). These embodiments produce effects of generating an inhibitor for binding of LOX-1 to oxLDL, an inhibitor for uptake of oxLDL into LOX-1-expressing cells, or a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a LOX-1-binding antibody which binds to any of LOX-1 proteins derived from a human, a rabbit, and a pig. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of a human, a rabbit, and a pig.

Other embodiments of the present invention provide LOX-1-binding antibodies which specifically bind to a LOX-1 protein, the antibodies having heavy chain CDR3 comprising one or more amino acid sequences selected from the group consisting of y) an amino acid sequence set forth in SEQ ID NO: 7, z) an amino acid sequence having 80% or more homology to the amino acid sequence y), a') an amino acid sequence having one or several amino acid deletions, substitutions, or additions in the amino acid sequence y), and b') an amino acid sequence encoded by a nucleotide sequence of a nucleic acid chain hybridizing under a stringent condition with a nucleic acid chain having a nucleotide sequence complementary to a nucleotide sequence encoding the amino acid sequence y). These embodiments produce effects of generating an inhibitor for binding of LOX-1 to oxLDL, an inhibitor for uptake of oxLDL into LOX-1-expressing cells, or a therapeutic or diagnostic agent for a cardiovascular disease, an atherosclerotic disease, thrombosis, thrombocytopenia, a systemic inflammatory response syndrome, an inflammation, or intimal hyperplasia of mammals.

Another embodiment of the present invention provides a LOX-1-binding antibody which binds to any of LOX-1 proteins derived from a human and a pig. This embodiment produces effects of generating a therapeutic or diagnostic agent for a cardiovascular disease, an atherosclerotic disease, thrombosis, thrombocytopenia, a systemic inflammatory response syndrome, an inflammation, or intimal hyperplasia of a human and a pig.

### EXAMPLES

Hereinafter, the present invention is further illustrated by Examples. However, the present invention is not limited to these Examples.

### < Example 1 : Preparation of recombinant LOX-1 and recombinant-LOX-1-expressing cells>

### (1-1) Production and purification of recombinant LOX-1

The following LOX-1 proteins were produced using pcDNA4/myc-HisA (Invitrogen, USA), which can be used for synthesizing the respective LOX-1 proteins as 6×Histidine tag (His tag)-fusion proteins. The LOX-1 proteins included recombinant human LOX-1 (amino acids 61-273; rhLOX-1) and a deletion mutant thereof (amino acids 137-273; delta Neck), recombinant mouse LOX-1 (amino acids 188-363 of mouse LOX-1; rmLOX-1), recombinant rabbit LOX-1 (amino acids 101-278 of rabbit LOX-1; rrLOX-1), and recombinant pig LOX-1 (amino acids 61-274 of pig LOX-1; rpLOX-1). The above production was carried out by using a procedure, the details of which is described in Sato et al., Atherosclerosis 2008 Oct., 200(2), 303-309, Epub 2008 Feb 12. The recombinant proteins were produced using a FreeStyle 293 Expression System (Invitrogen), and purified using a Probond protein purification kit (Invitrogen). The molecular weight of the proteins was determined by SDS-PAGE. The purified proteins were biotinylated by using a Biotin Labeling Kit-NH₂ (Dojindo, Japan). In addition, PCR primers used in this study were shown in Table 1.

[Table 1]

**Table 1. PCR primers used in this study.**

| Primer | Sequence |
|---|---|
| Leader-F | 5'-ATGCGGATCCGCCATGGCCTGGGCTCCTCTCCT |
| Leader-R | 5'-TGCCTGCACCAGGGAACCTG |
| hLOX-F | 5'-TCCCTGGTGCAGGCAGAAGAAGCTTCACAGGAG |
| hLOX-R | 5'-ATGCACCGGTCTGTGCTCTTAGGTTTGCC |
| hLOX-Neck-F | 5'-TCCCTGGTGCAGGCAGTAGCAAATTGTTCAGCTC |
| mLOX-F | 5'-TCCCTGGTGCAGGCAGAGTCCCAGAGAGAACTC |
| mLOX-R | 5'-ATGCACCGGTAATTTGCAAATGATTTGTC |
| rLOX-F | 5'-TCCCTGGTGCAGGCAGAGTCACAAAGGGAACTC |
| rLOX-R | 5'-ATGCACCGGTCTCTGATCTCAGCAGATTTG |
| pLOX-F | 5'-TCCCTGGTGCAGGCATCCCAGGTGTCTGATCTCCTG |
| pLOX-R | 5'-GACTACCGGTCTGTGCTCTCAAGAGATTCGC |

| | |
|---|---|
| Primers for generation of recombinant LOX-1 protein. Restriction sites are underlined. | |

### (1-2) SDS-PAGE of recombinant LOX-1

FreeStyle 293-F cells were used to produce the respective recombinant human LOX (rhLOX-1), recombinant mouse LOX (rmLOX-1), recombinant rabbit LOX (rrLOX-1), recombinant pig LOX (rpLOX-1), and delta Neck (a deletion mutant of rhLOX-1). These recombinant LOX-1 proteins were detected using SDS-PAGE under non-reducing conditions as bands of about 62 kDa, about 40 kDa, about 20 kDa, about 22 kDa, and about 30 kDa, respectively (FIG. 2A). In addition, the rhLOX-1 and delta Neck were also able to be detected using SDS-PAGE under reducing conditions as bands at molecular weights that were half the molecular weights of the above bands. This result suggests that the rhLOX-1 and the delta Neck are each crosslinked by themselves at Cys 140 via a disulfide bond.

In addition, proteins exhibiting an activity of binding to human oxLDL failed to demonstrate an activity of binding to LDL, a negative control (FIG. 2B). This result verifies that the recombinant proteins have proper structures and functions. The rmLOX-1, rrLOX-1, and rpLOX-1 formed monomers. Thus, the same bands were detected under either reducing conditions or non-reducing conditions. Either a broad band or two bands were detected for the recombinant LOX-1 proteins (i.e., human, mouse, rabbit, and pig LOX-1)(FIG. 2A). These differences in their molecular weight may seem to be due to sugar modifications because LOX-1 contains putative N-glycosylation signals.

Also, FIG. 2 shows a profile obtained by SDS-PAGE and activities of the recombinant LOX-1 proteins. FIG. 2A shows a profile obtained by SDS-PAGE of rhLOX-1 (lane 1), delta Neck (lane 2), rmLOX-1 (lane 3), rrLOX-1 (lane 4), and rpLOX-1 (lane 5). The recombinant proteins were purified from supernatants of 293-F cells by nickel affinity chromatography. All the samples were subjected to SDS-PAGE under non-reducing conditions, and were stained with CBBR. The numbers in the left side denote molecular weights (kDa). FIG. 2B shows respective activities of binding of rhLOX-1 and delta Neck to oxLDL (black), LDL (a negative control, white), and BSA (control, gray). The activities were determined by an ELISA using a plate coated with biotin-labeled rhLOX-1 or a plate coated with biotin-labeled delta Neck. Data were shown with standard deviation of three independent experiments.

### <Example 2 : Construction of phage display and LOX-1 antibody acquisition>

### (2-1) Construction of phage display library of chicken scFv mAbs against rhLOX-1

Chicken scFv mAbs were generated by using phage display utilizing chicken materials. The above was carried out in accordance with a procedure, the details of which are described in Nakamura et al., J Vet Med Sci. 2004 Jul., 66(7), 807-814. An inbred H-B15 chicken was intraperitoneally immunized with rhLOX-1 (50 µg/ml/chicken) containing an equivalent alum solution (ALUM) for one month. Then, the chicken received, with a three-week interval, three additional intraperitoneal injections of the immunogen which combined the same antigen and ALUM. Four days after the final injection, spleen cells were isolated from the immunized chicken. After that, RNA was extracted from the spleen cells, and immunoglobulin variable region (V_{H} and V_{L}) genes were amplified. Finally, an scFv phage library was constructed.

### (2-2) Selection using panning against rhLOX-1 or rmLOX-1

An scFv library obtained using rhLOX-1-immunized-chicken-derived phage display was subjected to panning against rhLOX-1 or rmLOX-1. In order to select an rhLOX-1-specific antibody, 100 µl (10 µg/ml) of rhLOX-1 was coated on a Maxisorp Nunc-Immuno module (NUNC, USA). ELISA plates were beforehand blocked with 2% (w/v) non-fat dried milk powder (EuroClon, Italia) in a phosphate-buffered saline (PBS) for 1 hour at room temperature. In order to select against rmLOX-1, 100 µl (5 µg/ml) of biotin-labeled rmLOX-1 was coated on Nunc Immobilizer Streptavidin plates (NUNC). Panning selection was performed using a procedure described in Nakamura et al., J Vet Med Sci. 2004 Jul., 66(7), 807-814.

### (2-3) Construction of recombinant chicken IgY (rIgY) and chicken-human chimeric IgG4 (chimeric IgG) antibodies

By using V_{H} and V_{L} genes derived from chicken antibodies which were obtained using the phage display technique in the above experiments, chicken anti-LOX-1 rIgY and chicken-human chimeric anti-LOX-1 IgG were generated. At that occasion, a plasmid vector was used to construct the respective light and heavy chains in accordance with a procedure described in Shimamoto et al., Biologicals 2005 Sep., 33(3), 169-74, and Nishibori et al., Mol Immunol. 2006 Feb., 43(6), 634-42. Constructed plasmid DNAs were transfected using a FuGENE HD transfection reagent into COS-7 cells or FreeStyle 293-F cells (Invitrogen).

### (2-4) Detection of recombinant LOX-1

The recombinant LOX-1 proteins were detected by Western blotting. The recombinant LOX-1 proteins were subjected to SDS-PAGE under non-reducing conditions, and were transferred onto an Immun-Blot PVDF membrane (Bio-Rad, USA) for 1 hour at a current of 350 mA. The above membrane was incubated in the presence of a chicken anti-LOX-1 rIgY for 1 hour at room temperature, and the recombinant proteins were detected using ECL plus (GE Healthcare, UK). Chemiluminescent signals were analyzed with LAS-3000 (Fuji Film, Japan). The chicken anti-LOX-1 rIgY and the chicken-human chimeric anti-LOX-1 IgG were detected by Western blotting using a procedure described in Shimamoto et al., Biologicals 2005 Sep., 33(3), 169-174 and Sato et al., Atherosclerosis 2008 Oct., 200(2), 303-309, Epub 2008 Feb 12. A horseradish-peroxidase (HRP)-labeled anti-chicken-IgG Fab fragment (Bethyl, USA) was used for detecting the chicken anti-LOX-1 rIgY and the chicken-human chimeric anti-LOX-1 IgG. In order to detect the chicken-human chimeric anti-LOX-1 IgG, a mouse anti-human-IgG4 antibody (BD Biosciences, USA) was used as a primary antibody, and an HRP-labeled anti-mouse-IgG antibody (Southern Biotech, USA) was used as a secondary antibody. The chicken anti-LOX-1 rIgY and the chicken-human chimeric anti-LOX-1 IgG were detected using ECL plus and LAS-3000.

As described above, by using spleen cells of a chicken immunized with rhLOX-1, an scFv phage library (5.0×10⁸ cfu) was constructed. After six rounds of selection using panning against rhLOX-1, a concentration specificity of the scFv phage library was detected by an ELISA. Among 207 phage clones derived from the library following 5 or 6 rounds of panning, 113 clones were reacted with rhLOX-1. As a result of nucleic acid sequence analysis of positive clones, it was demonstrated that these clones were able to be classified into 50 independent subclasses of clones. For selection using panning against rmLOX-1 by utilizing a similar phage display library, two independent clones derived from the library following 5 or 6 rounds of panning were selected. As a result, a total of 52 clones were established as rIgY for quantitative tests.

### <Example 3: Evaluation of rIgY cross-reactivity>

### (3-1) Evaluation of rIgY cross-reactivity by ELISA

Wells of a Nunc Immobilizer Streptavidin plate were coated in the presence of a carbonate buffer (pH 9.5) for 1 hour at room temperature with 50 µl (1 µg/ml) of respective biotin-labeled rhLOX-1, delta Neck, rmLOX-1, rrLOX-1, rpLOX-1, and BSA (as a control antigen). After the wells were washed with PBS-T, 1 µg/ml of the chicken anti-LOX-1 rIgY was added to the respective wells. The plate was incubated at 37°C for 1 hour. After the wells were washed with PBS-T, bound antibodies were detected using an HRP-labeled goat anti-chicken IgG (H+L) (Kirkegaard and Perry Laboratories, USA). After the wells were washed with PBS-T, o-phenylene diamine sulfate (OPD, Sigma, USA) was added, and absorbance at 490 nm was measured using a Model 680 microplate reader (Bio-Rad). A human anti-human-LOX-1 mAb TS-92 [Hu et al., Biochem Biophys Res Commun. 2003 Aug 8, 307(4), 1008-1012] was used as a positive control.

### (3-2) Preparation of cells expressing human, rabbit, or pig recombinant LOX-1

A cDNA encoding human LOX-1 was subcloned into a Tet-On Gene Expression vector pTRE2hyg (Clontech Laboratories, USA). The plasmid was transfected into CHO-K1 Tet-On cells (Clontech Laboratories) by using a Lipofectamin 2000 transfection reagent (Invitrogen) in accordance with the manufacturer's protocol. Stable transformants were selected in the presence of Ham's F12/10% fetal bovine serum (FBS) supplemented with 400 µg/ml hygromycin B (Wako, Japan). Then, cells which expressed LOX-1 in response to doxycycline (Wako) were selected. Before the examination, LOX-1 expression was induced with 1 µg/ml of doxycyline for 24 hours. The cDNAs of rabbit and pig LOX-1 were each subcloned into a mammalian expression vector pEF6V5-HisA (Invitrogen). These plasmids were each transfected into CHO-K1 cells by using a FuGENE HD transfect reagent (Roche Diagnostics, Switzerland). Stable LOX-1-expressing cells were selected in the presence of Ham's F12/ 10% FBS supplemented with 8 µg/ml of blasticidin S (Invitrogen).

### (3-3) Evaluation of rIgY cross-reactivity by FACS analysis

The recombinant antibody and LOX-1-expressing CHO cells were together incubated at 4°C for 1 hour in the presence of PBS containing 0.1% FBS and 0.1% NaN₃ (a FACS buffer). After washing with the FACS buffer, the cells were incubated at 4°C for 30 minutes with an FITC-labeled anti-chicken IgG (H+L) or an FITC-labeled anti-human IgG (H+L) (Southern Biotech, USA). Fluorescence was analyzed with FACS Calibur (BD, USA).

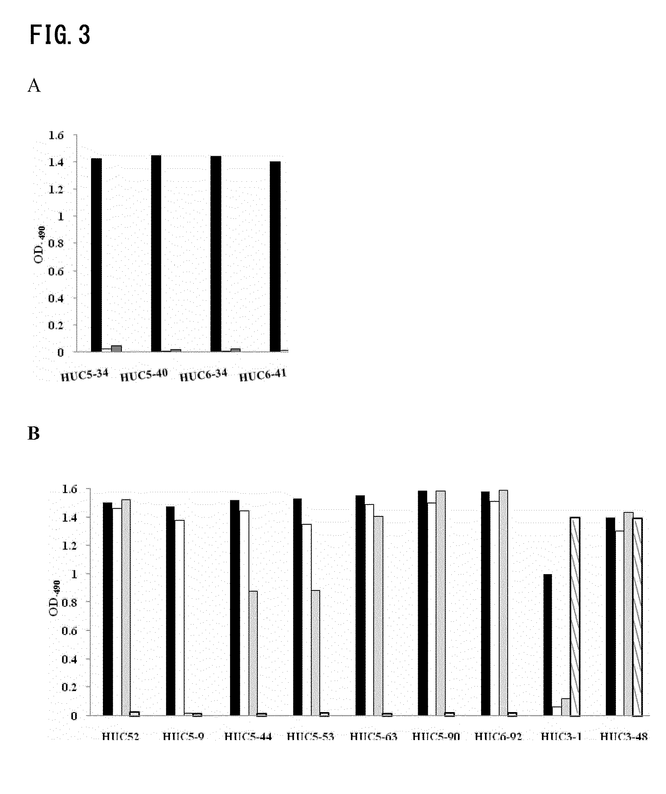

Fifty two chicken antibodies as obtained by s phage display technique were investigated, the antibodies recognizing the CTL or Neck domain in the recombinant LOX-1 proteins derived from different mammalian species as described above. The 52 rIgY antibodies against the recombinant LOX-1 were evaluated using an ELISA and FACS. Among the 52 clones, HUCS-34, HUCS-40, HUC6-34, and HUC6-41 reacted with rhLOX-1, but not with delta Neck (FIG. 3A). This result suggests that these four domain clones (anti-Neck-domain clones) can recognize the Neck domain of LOX-1. The rest of clones reacted with both rhLOX-1 and delta Neck at a similar intensity, which indicates these clones recognize the CTL domain. Among the 52 clones, 7 clones, including HUC52, HUCS-44, HUCS-53, HUCS-63, HUCS-90, HUC6-92, and HUC3-48, exhibited cross-reactivity to both rabbit LOX-1 and pig LOX-1 (FIG. 3B). HUCS-9 exhibited cross-reactivity to rpLOX-1 (FIG. 3B). Two clones HUC3-1 and HUC3-48, both of which had been selected using panning against rmLOX-1, reacted with rmLOX-1. Interestingly, HUC3-48 reacted with any of LOX-1 proteins derived from all the species used in the tests (FIG. 3B). According to the results of Western blotting, these clones failed to react with any of the recombinant LOX-1 derived from all the species used in the tests. This suggests that these clones recognize conformation of the epitope. In fact, these clones reacted with LOX-1-expressing cells as revealed by FACS analysis. Any of the clones tested did not react with BSA or wild-type CHO cells.

FIG. 3 shows rIgY antibody activities toward the recombinant LOX-1 proteins. FIG. 3A shows rIgY antibody activities toward hLOX-1 and delta NECK. The rIgYs (1 µg/ml) were added to wells coated with biotinylated rhLOX-1 (black), delta Neck (white), or BSA (a negative control). FIG. 3B shows cross-reactivity of 52 rIgY antibodies to the LOX-1 proteins derived from 4 different species. The rIgYs (1 µg/ml) were added to wells coated with biotinylated rhLOX-1 (black), rpLOX-1 (white), rrLOX-1 (gray) or rmLOX-1 (shaded). Among the 52 clones, 7 clones exhibited cross-reactivity to rhLOX-1 and/or rpLOX-1, and 2 clones recognized rmLOX-1.

### (3-4) Correlation between cross-reactivity and heavy chain CDR

In addition, Table 2 shows amino acid sequences of heavy chain CDR1, heavy chain CDR2, and heavy chain CDR3, which are regions determining specificity to an antigen, of the above HUC52, HUCS-9, HUCS-44, HUCS-53, HUCS-63, HUCS-90, HUC6-92, HUC3-1, and HUC3-48, all of which exhibited cross-reactivity. Of note is that the symbol "-" in Table 2 denotes a gap inserted so as to align the sequences.

[Table 2]

**Table 2. Amino acid sequences of CDRs**

| | CDR1 | CDR2 | CDR3 | 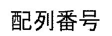 |
|---|---|---|---|---|
| HUC52 | DYGMG | VISSTGSYTNYGSAVK | GAYSSGY--DG--DGSS | 2, 3, 1 |
| HUC5-9 | DYGMG | EISAAGSTTFYGAAVK | CAYSSGY--DCGLSADI | 14, 15, 7 |
| HUC5-44 | SHNMG | SIS-TGSRTAYGAAVK | AADGWY---WDADIADL | 12, 13, 6 |
| HUC5-53 | SHNMG | SIS-TGSRTAYGAAVK | AADGWY---WDADIADL | 12, 13, 6 |
| HUC5-63 | SHNMG | SIS-TGSRTAYGAAVK | AADGWY---WDADIADL | 12, 13, 6 |
| HUC5-90 | DYGMG | VISSTGSYTNYGSAVK | CAYSSGY--DC--DGSS | 2, 3, 1 |
| HUC6-92 | DYGMG | VISSTGSYSNYGSAEK | CAYSSGY--DC--DGSS | 2, 16, 1 |
| HUC3-1 | SFNMF | AIRNDGSYTNYGAAVO | HV---AICTYGYCGAGW | 10, 11, 5 |
| HUC3-48 | SYAMQ | GIGYSGSSTWYVTAVK | GTGSGYCGSGSWCAG-S | 8, 9, 4 |

Among them, three antibodies (clones) including HUC52, HUCS-90, and HUC6-92 shared common properties that they had an identical heavy chain CDR3 sequence, CAYSSGYDCDGSS (SEQ ID NO: 1), and that they had cross-reactivity to human, rabbit, and pig LOX-1. In addition, three antibodies including HUC52, HUC5-90, and HUC6-92 shared a short sequence of heavy chain CDR1 sequence, DYGMG (SEQ ID NO: 2). Heavy chain CDR2 of HUC6-92 had two distinct amino acids. Except this difference, all of them had the identical sequences of the heavy chain CDR2.

Furthermore, three antibodies (clones) including HUC5-44, HUC5-53, and HUC5-63 shared common properties that they had an identical heavy chain CDR3 sequence, AADGWYWDADIADL (SEQ ID NO: 6), and that they had cross-reactivity to human, rabbit, and pig LOX-1. In addition, the three antibodies including HUC5-44, HUC5-53, and HUC5-63 shared a short sequence of a heavy chain CDR1 sequence, SHNMG (SEQ ID NO: 12). They also shared a heavy chain CDR2 sequence, SIS-TGSRTAYGAAVK (SEQ ID NO: 13).

Also, when amino acid sequence homology of heavy chain CDRs (i.e., heavy chain CDR1, heavy chain CDR2, and heavy chain CDR3) to the heavy chain CDRs of HUC52 was determined, HUC5-9 had 66.7% homology, HUC5-44 had 38.9% homology, HUC5-53 had 38.9% homology, HUC5-63 had 38.9% homology, HUC5-90 had 100%homology, HUC6-92 had 94.1% homology, HUC3-1 had 28.9% homology, and HUC3-48 had 21.6% homology. In a similar manner, when the homology of heavy chain CDRs of the rest 41 clones examined in the above experiments to the heavy chain CDRs of HUC52 was determined, all of them had 73.5% or less homology.

In addition, when amino acid sequence homology of heavy chain CDR3 to the CDR3 of HUC52 was determined, HUCS-9 had 60% homology, HUC5-44 had 6.7% homology, HUC5-53 had 6.7% homology, HUC5-63 had 6.7% homology, HUC5-90 had 100% homology, HUC6-92 had 100% homology, HUC3-1 had 0% homology, and HUC3-48 had 11.8% homology. In a similar manner, when the homology of heavy chain CDR3 of the rest 41 clones examined in the above experiments to the heavy chain CDR3 of HUC52 was determined, all of them had 56.5% or less homology.

In addition, when amino acid sequence homology of heavy chain CDR2 to the CDR2 of HUC52 was determined, HUC5-9 had 62.5% homology, HUC5-44 had 68.8% homology, HUC5-53 had 68.8% homology, HUC5-63 had 68.8% homology, HUC5-90 had 100% homology, HUC6-92 had 87.5% homology, HUC3-1 had 62.5% homology, and HUC3-48 had 50% homology. In a similar manner, when the homology of heavy chain CDR2 of the rest 41 clones examined in the above experiments to the heavy chain CDR2 of HUC52 was determined, all of them had 81.3% or less homology.

Also, amino acid sequences of heavy chain FRs (i.e., heavy chain FR1, heavy chain FR2, heavy chain FR3, and heavy chain FR4) were highly homologous among all the clones. For example, when the homology to the heavy chain FRs of HUC52 was determined, HUC5-9 had 92.3% homology, HUC5-44 had 91.2% homology, HUC5-53 had 91.2% homology, HUC5-63 had 91.2% homology, HUC5-90 had 100% homology, HUC6-92 had 98.9% homology, HUC3-1 had 91.2% homology, and HUC3-48 had 89.0% homology. In a similar manner, when the homology of heavy chain FRs of the rest 41 clones examined in the above experiments to the heavy chain FRs of HUC52 was determined, all of them had 86.8% or higher homology.

In addition, amino acid sequences of light chain CDRs (i.e., CDR1, light chain CDR2, and light chain CDR3) were less homologous among all the clones. For example, when the homology to the light chain CDRs of HUC52 was determined, HUC5-9 had 58.6% homology, HUC5-44 had 48.5% homology, HUC5-53 had 54.8% homology, HUC5-63 had 43.3% homology, HUC5-90 had 60.7% homology, HUC6-92 had 61.3% homology, HUC3-1 had 41.4% homology, and HUC3-48 had 43.3% homology. In a similar manner, when the homology of light chain CDRs of the rest 41 clones examined in the above experiments to the light chain CDRs of HUC52 was determined, all of them had 69.0% or less homology.

In addition, amino acid sequences of light chain CDR3 were less homologous among all the clones. For example, when the homology to the light chain CDR3 of HUC52 was determined, HUC5-9 had 41.7% homology, HUC5-44 had 25.0% homology, HUC5-53 had 58.3% homology, HUC5-63 had 33.3% homology, HUC5-90 had 54.5% homology, HUC6-92 had 54.5% homology, HUC3-1 had 25.0% homology, and HUC3-48 had 16.7% homology. In a similar manner, when the homology of light chain CDR3 of the rest 41 clones examined in the above experiments to the light chain CDR3 of HUC52 was determined, all of them had 70% or less homology.

### (3-5) Discussion of results

It is generally known that a variable region of heavy chain, a V_{H}, contributes largely to the specificity and affinity to an antigen. For example, a V_{H} gene has been extracted and amplified from a spleen of a mouse immunized with lysozyme. Next, the only V_{H} has been expressed solely. Then, it has been reported that the V_{H} fragment without a V_{L} has remarkably retained the affinity to the antigen [Ward et al., Nature 1989 Oct 12, 341(6242), 544-546].

It is generally considered that heavy chain CDRs of a V_{H} component are the most important regions of what determines antigen specificity, and heavy chain CDR3, in particular, plays a key role. The CDRs are regions which define specificity to an antigen. Accordingly, their amino acid sequences largely vary among antibodies. Thus, the CDRs are also referred to as hypervariable regions. Fv regions excluding the CDR are referred to as framework regions (FR), and are relatively well conserved among antibodies [Kabat et al., "Sequence of Proteins of Immunological Interest" US Dept. Health and Human Services, 1983].

In addition, with regard to currently commercially available antibody medicines, a strategy has been adopted that their antibody amino acid sequence is substituted by a human-derived sequence so as to decrease immunogenicity (toxicity) at the time of administration of the therapeutic agent to a human subject. The representative examples include a humanized antibody which can be constructed by grafting "only CDRs" of a mouse antibody to FV regions of a human antibody [Immunol. Today, 14, 243, 1993 and Int Rev Immunol 10, 241, 1993]. For example, humanized antibodies having generic names: Trastumab, Bevacizumab, Cetuximab, or Adalimumab etc. have been commercially available, and they account for significant share in the antibody medicine market. This delineates that the CDR regions are quite critical for characterizing antibody functions.

Then, the above experimental results indicate a correlation between reactivity of the respective clones and their amino acid sequence of heavy chain CDRs. Thus, it seems that in the anti-LOX-1 mAbs including HUC52, HUC5-9, HUC5-44, HUC5-53, HUC5-63, HUC5-90, HUC6-92, HUC3-1, and HUC3-48, the reactivity (the cross-reactivity and affinity) to the LOX-1 proteins is characterized by the amino acid sequence of the heavy chain CDR.

In addition, among heavy chain CDR1, heavy chain CDR2, and heavy chain CDR3, the amino acid sequence of the heavy chain CDR3 exhibited the highest correlation with the reactivity of the respective clones. Furthermore, the heavy chain CDR3 is identical between HUC6-92, and HUC52 and HUC5-90. However, the heavy chain CDR2 has two amino acid differences between them. Thus, it seems that the amino acid sequence of the heavy chain CDR3 characterizes the reactivity to the LOX-1 proteins. In addition, heavy chain CDR1 has only five amino acids. Consequently, if the reactivity is characterized only by the heavy chain CDR1, the amino acid sequence seems to be too short.

Also, since the homology of amino acid sequences of FRs (i.e., FR1, FR2, FR3, and FR4) were highly homologous among all the clones, FR amino acid sequences seem to be insignificant for reactivity of the anti-LOX-1 mAbs to LOX-1. Besides, amino acid sequences of light chain CDRs (i.e., light chain CDR1, light chain CDR2, and light chain CDR3) and light chain CDR3 are not shared among all the clones, so that the amino acid sequences of the light chain CDRs seem to be insignificant for reactivity of the anti-LOX-1 mAbs to LOX-1.

In addition, in view of the above facts, when an amino acid sequence excluding heavy chain CDRs of the LOX-1-mAbs or an amino acid sequence excluding heavy chain CDR3 of the LOX-1-mAbs is substituted by a sequence derived from another antibody, the reactivity of the LOX-1 mAbs seems to be able to remain the same. An advantageous effect of the substitution seems to be, for example, capable of decreasing immunogenicity in the case of administration of the therapeutic agent to a human subject by substituting by a sequence derived from a human. At this occasion, in order to increase reactivity of the anti-LOX-1 mAbs, the substituted region is preferably optimized by using a known process in the art (e.g., a phage display or a process for screening an antibody having high reactivity by mutating, at random, amino acid residues of the antibody). When FR regions are substituted, the FR regions seem to be able to be optimized by using, for example, FR shuffling [Damschroder et al., Mol Immunol. 2007 Apr., 44(11), 3049-3060, Epub 2007 Jan 22] or a process for substituting packaging residues and/or amino acid residues within a vernier zone [JP2006-241026A or Foote et al., J Mol Biol. 1992 Mar 20; 224(2):487-499].

### <Example 4 : Analysis of inhibition of binding of oxLDL to LOX-1 proteins by mAb>

### (4-1) Analysis of inhibition of binding of oxLDL to recombinant LOX-1 proteins by mAb

The inhibition was analyzed by a method according to Kakutani et al., Biochem Biophys Res Commun. 2001 Mar 23, 282(1), 180-185. Biotin-labeled rhLOX-1 (50 ng/well) was incubated and immobilized on a Nunc Immobilizer Streptavidin plate in the presence of 50 µl of a carbonate buffer at room temperature for 1 hour. After the plate was washed with PBS, rIgY (1 µg/ml), TS-92 (a positive control, 1 µg/ml), anti-2,4-dinitrophenyl (DNP) rIgY (a negative control, 1 µg/ml) [Sato et al., Atherosclerosis 2008 Oct., 200(2), 303-309, Epub 2008 Feb 12] were each added to wells coated with the rhLOX-1. Then, the plate was incubated at room temperature for 2 hours. After washed with PBS, the plate was incubated overnight at 4°C with 50 µl of human oxLDL (3 µg/ml) in the presence of PBS containing 20% (vol/vol) newborn calf serum (NBCS, Gibco, USA). After washed with PBS, the plate was incubated, so as to detect oxLDL, at room temperature for 2 hours with an HRP-labeled sheep anti-human-ApoB polyclonal antibody (The Binding Site, UK) that had been diluted 1000 times with 1% (w/v)-BSA-containing PBS. After washed with PBS, the plate was incubated at room temperature for 5 minutes with 50 µl of SureBlue Reserve TMB Microwell Peroxidase Substrate (Kirkegaard & Perry Laboratories, USA) to initiate a peroxidase reaction. The reaction was terminated by adding 0.1 M hydrochloric acid and 0.3 M sulfuric acid. The peroxidase activity was detected by measuring absorbance at a wavelength of 450 nm.

### (4-2) Analysis of inhibition of binding of oxLDL to LOX-1-expressing cells by mAb

LOX-1-expressing CHO cells were incubated at 37°C for 1 hour with an anti-LOX-1 antibody in the presence of 10%-NBCS-containing Ham's F12. After 1 hour, 1,1'-dioctadecyl-3,3,3',3'-tetramethylindocarbocyanine perchlorate (DiI, Molecular Probes, USA)-labeled oxLDL (3 µg/ml) was further added and incubated for 2 hours. The above cells were washed with PBS 3 times, and were fixed for 20 minutes with 4% (v/v) paraformaldehyde in PBS. The cell nuclei were counter-stained with 5 µg/ml of DAPI (Sigma), and were observed with a fluorescence microscope.

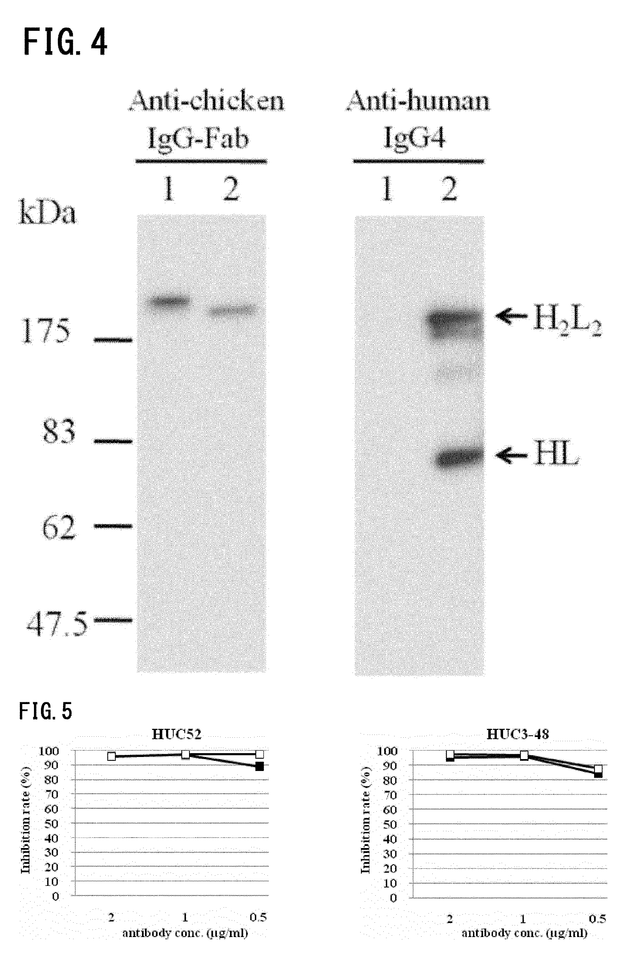

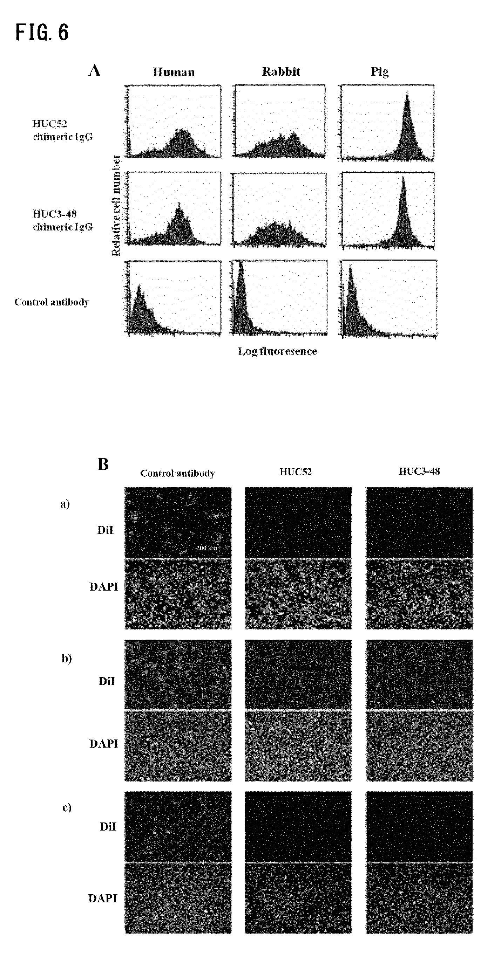

As described above, the neutralizing activity of 52 anti-LOX-1 antibodies was tested by a method according to Inoue et al., Circ Res. 2005 Jul 22, 97(2), 176-184, Epub 2005 Jun 16. Among the 52 clones, 44 clones exhibited the neutralizing activity. This suggests that the CTL domain essential in the oxLDL binding is recognized by the antibodies. The Neck domain of LOX-1 is not critical for the binding of oxLDL to LOX-1. However, HUC5-34, HUC5-40, HUC6-34, and HUC6-41 (FIG. 3A), all of which are anti-Neck-domain clones, inhibited the oxLDL binding to a small degree. The molecular weight (MW) of rhLOX-1 is about 62 kDa (FIG. 2A, lane 1), and the molecular weight of rIgY is about 250 kDa. Since the molecular weight of anti-Neck-domain clones is four times higher than the molecular weight of rhLOX-1, the anti-Neck-domain clones may be likely to impose steric hindrance. Chimeric IgG antibodies were reconstructed by using two clones (i.e., HUC52 having cross-reactivity to rabbit and pig LOX-1, and HUC3-48 recognizing LOX-1 proteins derived from all the species used in the experiments). The IgG4 subclass was selected so as to circumvent antibody-dependent cellular cytotoxicity (ADCC) and Ig effector functions accompanying complement activation. These chimeric antibodies behaved as their original rIgY forms exhibited a similar reaction (FIG. 5). The inhibition experiments were carried out by using LOX-1-expressing CHO cells. Whereas tests were involved with different species, these two chimeric IgG reacted with LOX-1-expressing CHO cells (FIG. 6A), and blocked the binding of oxLDL to the LOX-1-expressing CHO cells (FIG. 6B). In a similar manner, HUC3-48 exhibited an inhibitory activity toward mouse-LOX-1-expressing cells.

Meanwhile, FIG. 4 shows results of Western blotting of a recombinant chicken antibody and a chicken-human chimeric antibody. The rIgY (lane 1) and the chimeric IgG (lane 2) were subjected to 10% SDS-polyacrylamide gel electrophoresis under non-reducing conditions. An anti-chicken-IgG-Fab antibody and an anti-human-IgG4 antibody were used for detection of the above antibodies. The numbers in the left side denote molecular weights (kDa). The "H₂L₂" indicates a complete antibody, and the "HL" indicates an antibody at a molecular weight that is half the molecular weight of the complete antibody.

FIG. 5 shows an inhibition of binding of oxLDL to LOX-1 by anti-LOX-1 antibodies. The rhLOX-1 was used as a capturing molecule. The symbols "rIgY (closed square)" and "chimeric IgG (open square)" denoted a ratio of inhibiting the binding of oxLDL to LOX-1. An anti-human-ApoB antibody was used. An anti-DNP rIgY was used as a negative control, and was used for comparison as a standard.

FIG. 6 shows reactivity and blocking activity of the chimeric IgG toward LOX-1-expressing cells. In FIG. 6A, LOX-1-expressing CHO cells were incubated with the chimeric IgG (1 µg/ml), and were detected by using an FITC-labeled anti-human-IgG antibody. Fluorescence was analyzed by FACS. In FIG. 6B, CHO cells expressing each LOX-1 derived from human a), rabbit b), and pig c) were incubated with the chimeric IgG (5 µg/ml). DiI-labeled oxLDL (3 µg/ml) was added and incubated for 2 hours. Then, the cells were fixed with 4% (v/v) paraformaldehyde. After that, the cells were stained with DAPI, and were observed with a fluorescence microscope.

### (4-3) Discussion of results

The above HUC52 and HUC3-48 reacted with LOX-1-expressing CHO cells, and exhibited an activity of inhibiting the binding of oxLDL to LOX-1-expressing cells. This indicates that the above clones seem to be effective in prevention or treatment of diseases such as a cardiovascular disease, an atherosclerotic disease, thrombosis, thrombocytopenia, a systemic inflammatory response syndrome, an inflammation, and intimal hyperplasia which are involved with LOX-1. This is because anti-LOX-1 mAbs having a function of inhibiting the binding of LOX-1 to oxLDL have been demonstrated to have a therapeutic effect on a cardiovascular disease, an atherosclerotic disease, thrombosis, thrombocytopenia, a systemic inflammatory response syndrome, an inflammation, or intimal hyperplasia of mammals, which is reported in WO01/064862; Inoue et al., Circ Res. 2005 Jul 22, 97(2), 176-184; Hinagata et al., Cardiovasc Res. 2006 Jan., 69(1), 263-271; and Honjo et al., Proc Natl Acad Sci USA. 2003 Feb 4, 100(3), 1274-1279.

Further, HUC5-9, HUC5-44, HUC5-53, HUC5-63, HUC5-90, HUC6-92, and HUC3-1 were constructed in the same manner as for HUC52 and HUC3-48, and a similar binding capability of them was verified by an ELISA (FIG. 3). It is supposed that they react with the LOX-1-expressing CHO cells and exhibit the activity of inhibiting the binding of oxLDL to the LOX-1-expressing cells. Because of this, HUC5-9, HUC5-44, HUC5-53, HUC5-63, HUC5-90, HUC6-92, and HUC3-1 seems to be able to be suitably used for applications as a therapeutic and/or diagnostic agent for the above diseases.

In addition, according to the above experimental results, nine mAbs (i.e., HUC52, HUC5-9, HUC5-44, HUC5-53, HUC5-63, HUC5-90, HUC6-92, HUC3-1, and HUC3-48) were obtained which specifically bound to human LOX-1 and which had cross-reactivity to one or two or all of pig LOX-1, rabbit LOX-1, and mouse LOX-1. Any of the nine mAbs (i.e., nine clones) having such cross-reactivity seems to be a useful mAb for development of a therapeutic and/or diagnostic agent. When the cross-reactivity is emphasized, HUC-3-1 and HUC3-48 which have cross-reactivity to mouse LOX-1 seem to be particularly useful mAbs among the nine mAbs. This is because mice are readily available and are easily handled, so that mice remain the most frequently used animal model for various human diseases. Mice are important in analyzing disease manifestation and verifying effects of a therapeutic or diagnostic agent. In addition, mice are also important disease animal model for a human cardiovascular disease or atherosclerotic disease, etc., which is involved with LOX-1. For example, an ApoE-knockout mouse is being used as an animal model for primary hyperlipidemia and used for analyzing LOX-1 functions [Sato et al., Atherosclerosis, 2008 Oct., 200(2), 303-309, Epub 2008 Feb 12]. In view of the above, when the cross-reactivity is emphasized, anti-LOX-1 mAbs exhibiting cross-reactivity to human and mouse LOX-1 seem to be particularly suitably used for development of a novel therapeutic and/or diagnostic agent for diseases such as a cardiovascular disease and an atherosclerotic disease.

### <Example 5 : Evaluation of capability of binding of anti-LOX-1 mAbs to LOX-1 by using BIACORE>

### (5-1) Evaluation of capability of binding of HUC52 to LOX-1

An association rate constant (ka), a dissociation rate constant (kd) and a dissociation constant (K_{D}) of HUC52 for human LOX-1 were determined using a commercially available measurement kit, BIACORE X (GE Healthcare Bio-Sciences, Ltd.). It is notable that the HUC52 was reconstructed as a human-chicken chimeric antibody prior to its measurement.

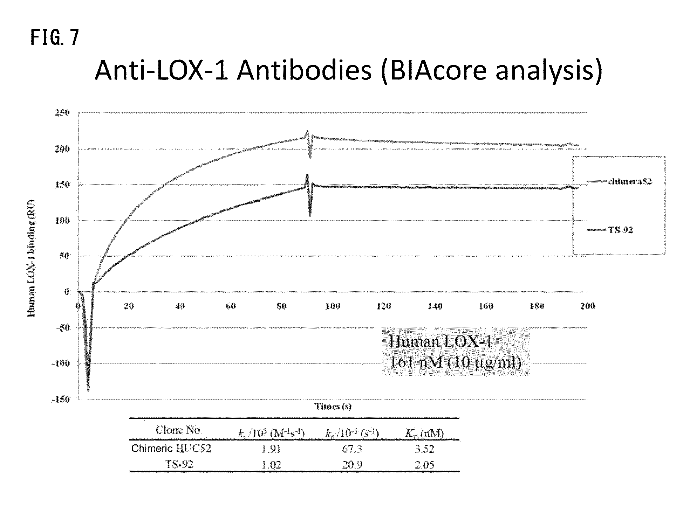

The manipulations were carried out in accordance with the manufacturer's protocol and experimental procedure attached to the above kit. Human LOX-1 was immobilized on a sensor chip. HUC52 was applied into a flow cell to carry out analysis. Antibody binding was measured over time to yield a sensorgram. Based on the resulting sensorgram, an association rate constant (ka), a dissociation rate constant (kd) and a dissociation constant (K_{D} ; K_{D} = kd/ka) were calculated using analysis software (BIAevaluation 3.0) attached to the kit. FIG. 7 shows a result comparing to an anti-human-LOX-1 antibody TS-92 [Hu et al., Biochem Biophys Res Commun. 2003 Aug 8, 307(4), 1008-12].

### (5-2) Discussion of results

HUC52 and TS-92 have association rate constants (ka) of 1.91 x 10⁵ M⁻¹s⁻¹ and 1.02 x 10⁵ M⁻¹s⁻¹, respectively. The HUC52 exhibits a markedly higher value. In addition, according to response curves ranging from the reaction initiation to 90 seconds for HUC52 and TS-92 in FIG. 7, it can be understood that HUC52 exhibits a steeper upward curve. These data demonstrates that HUC52 had a much faster binding speed than TS-92, and abundantly bound to human LOX-1.

In addition, the dissociation rate constants (kd) of HUC52 and TS-92 are 67.3 x 10⁻⁵ s⁻¹ and 20.9 x 10⁻⁵ s⁻¹, respectively. The dissociation constants (K_{D}) of HUC52 and TS-92 are 3.52 nM and 2.05 nM, respectively, either of which is a low number. In addition, compared to the response curves ranging from the reaction initiation to 90 seconds in FIG. 7, it can be understood that response curves encompassing after 90 seconds following the reaction initiation for any of HUC52 and TS-92 similarly exhibit a slight downward curve. These data demonstrate that dissociation of HUC52 and TS-92 from human LOX-1 is mild, and the dissociation amount is low.

HUC52 exhibits a much higher association rate constant (ka) than TS-92 of an anti-human-LOX-1 antibody. This suggests that HUC52 can be suitably used for applications as a therapeutic and/or diagnostic agent for diseases such as a cardiovascular disease and an atherosclerotic disease which are involved with LOX-1. This is because if the association rate constant (ka) is large, the anti-LOX-1 antibody more efficiently reacts with LOX-1. Thus, when the anti-LOX-1 antibody is, for example, developed for applications as a therapeutic agent and is sold, its usage can be made low. In addition, effects can be achieved which reduce development cost, user's financial burden, and patient's physical strain. The same applies to development and sales of other applications such as a diagnostic agent. Also, the experimental results of Example 4 demonstrate that HUC52 has cross-reactivity to human, rabbit, and pig LOX-1. Accordingly, HUC52 seems to be useful for applications as a therapeutic and/or diagnostic agent.

In addition, HUC5-9, HUC5-44, HUC5-53, HUC5-63, HUC5-90, HUC6-92, HUC3-1, and HUC3-48 were constructed in the same manner as for HUC52, and a similar binding capability of them was verified by an ELISA (FIG. 3). It is supposed that they have a high association rate constant (ka) for LOX-1. Because of this, HUC5-9, HUC5-44, HUC5-53, HUC5-63, HUC5-90, HUC6-92, HUC3-1, and HUC3-48 seem to be able to be suitably used for applications as a therapeutic and/or diagnostic agent.

### <Discussion of results>

According to the above experimental results of Examples 1 to 6, nine mAbs were obtained which specifically bound to human LOX-1 and which had cross-reactivity to one or two or all of pig, rabbit, and mouse LOX-1. This demonstrates that anti-LOX-1 mAbs have been obtained which can be suitably used for applications as a therapeutic and/or diagnostic agent for diseases such as a human cardiovascular disease and atherosclerotic disease which are involved with LOX-1. In addition, when the cross-reactivity is emphasized, HUC-3-1 and HUC3-48 having cross-reactivity to human and mouse LOX-1 seem to be particularly suitably used.

Furthermore, according to the above experimental results of Examples 1 to 6, nine anti-LOX-1 mAbs (i.e., HUC52, HUC5-9, HUC5-44, HUC5-53, HUC5-63, HUC5-90, HUC6-92, HUC3-1, HUC3-48) having a high association rate constant (ka) for LOX-1 have been obtained. This demonstrates that anti-LOX-1 mAbs have been obtained which can be suitably used for applications as a human, pig, rabbit, or mouse therapeutic and/or diagnostic agent for diseases such as a cardiovascular disease and atherosclerotic disease which are involved with LOX-1.

When used as a therapeutic agent, the anti-LOX-1 mAbs exert an effect of inhibiting the binding of oxLDL to LOX-1 or an effect of inhibiting the uptake of oxLDL into LOX-1-expressing cells. This seems to achieve therapeutic effects. For example, anti-LOX-1 mAbs have been demonstrated in an *in vivo* experiment to have a therapeutic effect on a cardiovascular disease, an atherosclerotic disease, thrombosis, thrombocytopenia, a systemic inflammatory response syndrome, an inflammation, or intimal hyperplasia of mammals, which is reported in WO01/064862; Inoue et al., Circ Res. 2005 Jul 22, 97(2), 176-184; Hinagata et al., Cardiovasc Res. 2006 Jan., 69(1), 263-271; and Honjo et al., Proc Natl Acad Sci USA. 2003 Feb 4, 100(3), 1274-1279.

In addition, when the anti-LOX-1 mAbs are used as a diagnostic agent, it seems that the above diseases can be diagnosed by measuring and comparing the levels of the LOX-1 expression in, for example, a standard cell, a test cell, a test serum, or a pathologic cell of any of the above diseases in accordance with the binding mode between the anti-LOX-1 mAbs and LOX-1. For example, according to Hayashida et al., Circulation 2005 Aug 9, 112(6), 812-8, Epub 2005 Aug 1, a soluble form of LOX-1 (sLOX-1) released from serum derived from a patient with an acute coronary syndrome (ACS) by protein cleavage has been detected. This serum sLOX-1 levels are determined using anti-LOX-1 mAbs, which is likely to allow for diagnosis of the ACS.

As described above, the present invention has been illustrated based on the Examples. These Examples are just examples. It should be understood by those skilled in the art that various modifications are possible and such modifications are also within the scope of the present invention.

## Claims

1. An anti-LOX-1 antibody which specifically binds to a LOX-1, comprising heavy chain CDR3 comprising one or more amino acid sequences selected from the group consisting of:
a) an amino acid sequence set forth in SEQ ID NO: 1;
b) an amino acid sequence having 80% or more homology to the amino acid sequence a);
c) an amino acid sequence having one or several amino acid deletions, substitutions, or additions in the amino acid sequence a); and
d) an amino acid sequence encoded by a nucleotide sequence of a nucleic acid chain hybridizing under a stringent condition with a nucleic acid chain having a nucleotide sequence complementary to a nucleotide sequence encoding the amino acid sequence a).

2. The anti-LOX-1 antibody according to Claim 1, further comprising:
heavy chain CDR1 comprising one or more amino acid sequences selected from the group consisting of:
e) an amino acid sequence set forth in SEQ ID NO: 2;
f) an amino acid sequence having 90% or more homology to the amino acid sequence e);
g) an amino acid sequence having one amino acid deletion, substitution, or addition in the amino acid sequence e); and
h) an amino acid sequence encoded by a nucleotide sequence of a nucleic acid chain hybridizing under a stringent condition with a nucleic acid chain having a nucleotide sequence complementary to a nucleotide sequence encoding the amino acid sequence; and
heavy chain CDR2 comprising one or more amino acid sequences selected from the group consisting of:
i) an amino acid sequence set forth in SEQ ID NO: 3;
j) an amino acid sequence having 85% or more homology to the amino acid sequence i);
k) an amino acid sequence having one or several amino acid deletions, substitutions, or additions in the amino acid sequence i); and
l) an amino acid sequence encoded by a nucleotide sequence of a nucleic acid chain hybridizing under a stringent condition with a nucleic acid chain having a nucleotide sequence complementary to a nucleotide sequence encoding the amino acid sequence i).

3. The anti-LOX-1 antibody according to Claim 1, wherein the antibody binds to a LOX-1 derived from mammals of different species.

4. The anti-LOX-1 antibody according to Claim 1, wherein the antibody binds to any of LOX-1s derived from a human, a rabbit, and a pig.

5. The anti-LOX-1 antibody according to Claim 1, wherein the antibody is selected from the group consisting of a chicken antibody, a chicken-human chimeric antibody, and a humanized antibody.

6. The anti-LOX-1 antibody according to Claim 5, wherein a subclass of the chicken-human chimeric antibody or the humanized antibody is IgG4.

7. The anti-LOX-1 antibody according to Claim 1, wherein the antibody specifically binds to a CTL domain of the LOX-1.

8. The anti-LOX-1 antibody according to Claim 1, wherein the antibody binds to a wild-type or mutated LOX-1.

9. The anti-LOX-1 antibody according to Claim 1, wherein the antibody is an antibody fragment.

10. The anti-LOX-1 antibody according to Claim 1, wherein the antibody is a monoclonal antibody.

11. A polynucleotide, comprising a nucleotide sequence encoding the anti-LOX-1 antibody according to Claim 1.

12. A vector, comprising the polynucleotide according to Claim 11 or a portion thereof.

13. An inhibitor for binding of LOX-1 to oxidized LDL, comprising the anti-LOX-1 antibody according to Claim 1.

14. An inhibitor for uptake of oxidized LDL into LOX-1-expressing cells, comprising the anti-LOX-1 antibody according to Claim 1.

15. A therapeutic agent, comprising the anti-LOX-1 antibody according to Claim 1,
wherein the agent is used for treating a cardiovascular disease, an atherosclerotic disease, thrombosis, thrombocytopenia, a systemic inflammatory response syndrome, an inflammation, or intimal hyperplasia of mammals.

16. The therapeutic agent according to Claim 15, wherein the mammals are any of organisms from a human, a rabbit, and a pig.

17. A diagnostic agent, comprising the anti-LOX-1 antibody according to Claim 1,
wherein the agent is used for diagnosing a cardiovascular disease, an atherosclerotic disease, thrombosis, thrombocytopenia, a systemic inflammatory response syndrome, an inflammation, or intimal hyperplasia of mammals.

18. The diagnostic agent according to Claim 17, wherein the mammals are any of organisms from a human, a rabbit, and a pig.

19. A anti-LOX-1 antibody which specifically binds to a LOX-1, comprising heavy chain CDR3 comprising one or more amino acid sequences selected from the group consisting of:
m) an amino acid sequence set forth in SEQ ID NO: 4;
n) an amino acid sequence having 80% or more homology to the amino acid sequence m);
o) an amino acid sequence having one or several amino acid deletions, substitutions, or additions in the amino acid sequence m); and
p) an amino acid sequence encoded by a nucleotide sequence of a nucleic acid chain hybridizing under a stringent condition with a nucleic acid chain having a nucleotide sequence complementary to a nucleotide sequence encoding the amino acid sequence m).

20. The anti-LOX-1 antibody according to Claim 19, wherein the antibody binds to any of LOX-1 s derived from a human, a mouse, a rabbit, and a pig.

21. A anti-LOX-1 antibody which specifically binds to a LOX-1, comprising heavy chain CDR3 comprising one or more amino acid sequences selected from the group consisting of:
q) an amino acid sequence set forth in SEQ ID NO: 5;
r) an amino acid sequence having 80% or more homology to the amino acid sequence q);
s) an amino acid sequence having one or several amino acid deletions, substitutions, or additions in the amino acid sequence q); and
t) an amino acid sequence encoded by a nucleotide sequence of a nucleic acid chain hybridizing under a stringent condition with a nucleic acid chain having a nucleotide sequence complementary to a nucleotide sequence encoding the amino acid sequence q).

22. The anti-LOX-1 antibody according to Claim 21, wherein the antibody binds to any of LOX-1s derived from a human and a mouse.

23. A anti-LOX-1 antibody which specifically binds to a LOX-1, comprising heavy chain CDR3 comprising one or more amino acid sequences selected from the group consisting of:
u) an amino acid sequence set forth in SEQ ID NO: 6;
v) an amino acid sequence having 80% or more homology to the amino acid sequence u);
w) an amino acid sequence having one or several amino acid deletions, substitutions, or additions in the amino acid sequence u); and
x) an amino acid sequence encoded by a nucleotide sequence of a nucleic acid chain hybridizing under a stringent condition with a nucleic acid chain having a nucleotide sequence complementary to a nucleotide sequence encoding the amino acid sequence u).

24. The anti-LOX-1 antibody according to Claim 23, wherein the antibody binds to any of LOX-1s derived from a human, a rabbit, and a pig.

25. An Anti-LOX-1 antibody which specifically binds to a LOX-1, comprising heavy chain CDR3 comprising one or more amino acid sequences selected from the group consisting of:
y) an amino acid sequence set forth in SEQ ID NO: 7;
z) an amino acid sequence having 80% or more homology to the amino acid sequence y);
a') an amino acid sequence having one or several amino acid deletions, substitutions, or additions in the amino acid sequence y); and
b') an amino acid sequence encoded by a nucleotide sequence of a nucleic acid chain hybridizing under a stringent condition with a nucleic acid chain having a nucleotide sequence complementary to a nucleotide sequence encoding the amino acid sequence y).

26. The anti-LOX-1 antibody according to Claim 25, wherein the antibody binds to any of LOX-1s derived from a human and a pig.
